(19) Europäisches Patentamt
European Patent Office
Office européen des brevets

(11) Veröffentlichungsnummer : **0 014 966**
**B1**

(12) **EUROPÄISCHE PATENTSCHRIFT**

(45) Veröffentlichungstag der Patentschrift : **05.06.85**

(21) Anmeldenummer : **80100756.8**

(22) Anmeldetag : **14.02.80**

(51) Int. Cl.⁴ : **C 07 D307/94**, C 07 C 59/90, C 07 C 59/215, C 07 C 49/737, A 61 K 31/365, A 61 K 31/19, A 61 K 31/12// C07C49/517, C07C49/513, C07C49/753, C07C43/196, C07C69/145, C07C69/16, C07C69/14, C07C35/37, C07D317/72, C07D493/10

(54) **Neue deA-Steroide, ihre Herstellung und deA-Steroide zur Verwendung als Pharmazeutika.**

(30) Priorität : **23.02.79 CH 1838/79**
**10.01.80 CH 172/80**

(43) Veröffentlichungstag der Anmeldung : **03.09.80 Patentblatt 80/18**

(45) Bekanntmachung des Hinweises auf die Patenterteilung : **05.06.85 Patentblatt 85/23**

(84) Benannte Vertragsstaaten : **AT BE CH DE FR GB IT LU NL SE**

(56) Entgegenhaltungen :
**US-A- 2 971 009**
**US-A- 3 412 107**
**JOURNAL OF ORGANIC CHEMISTRY, Band 26, Nr. 10, 24. Oktober 1961, Washington, US, L.J. CHINN et al.: "Studies in the total synthesis of steroids and their analogs. II. Des-A Analogs of Clinically active steroids" Seiten 3910-3915**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**
**CH-4002 Basel (CH)**

(72) Erfinder : **Fürst, Andor, Dr.**
**Magnolienpark 14**
**CH-4052 Basel (CH)**
Erfinder : **Keller, Peter, Dr.**
**Hinterlindenweg 53**
**CH-4153 Reinach (CH)**
Erfinder : **Müller, Marcel, Dr.**
**Quellenweg 10**
**CH-4402 Frenkendorf (CH)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 014 966 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue deA-Steroide, ein Verfahren zu ihrer Herstellung sowie deA-Steroide zur Verwendung als Pharmazeutika.

Die deA-Steroide zur Verwendung als Pharmazeutika besitzen die allgemeine Formel

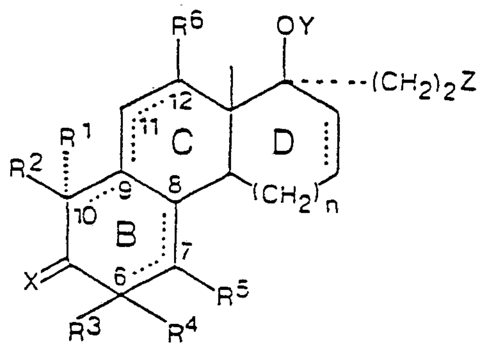

(I)

worin $R^1$ und $R^2$ Wasserstoff oder $C_{1-5}$-Alkyl ; $R^3$ Wasserstoff, Methyl oder Halogen ; $R^4$ Wasserstoff oder, falls $R^3$ Methyl ist, Wasserstoff oder Methyl ; $R^5$ Wasserstoff, Cyano, $C_{1-5}$-Alkoxy, oder einen Rest $SR^7$ ; oder $R^3$ und $R^5$ zusammen Methylen ; $R^6$ Wasserstoff oder $C_{1-7}$-Alkanoylthio ; $R^7$ $C_{1-7}$-Alkanoyl, $C_{1-8}$-Alkyl, Benzyl oder Phenäthyl ; X Oxo oder Methylen ; Y Wasserstoff ; Z Hydroxymethyl oder eine Gruppe —COA ; A Hydroxy ; oder Y und A zusammen O—C-Bindung bedeuten ; n 0 oder 1 ist und die punktierten ringständigen Bindungen fakultativ sind : wobei jeder Ring höchstens eine Doppelbindung enthalten soll ; der Ring B 9(10)-ungesättigt ist, wenn der Ring C 11(12)-ungesättigt ist ; mindestens ein Rest $R^5$ oder $R^6$ Wasserstoff ist ; $R^5$ Wasserstoff ist, wenn $R^4$ Methyl ist ; $R^5$ Wasserstoff ist oder $R^3$ und $R^5$ zusammen Methylen sind, und $R^6$ Wasserstoff ist, wenn Z Carboxy ist ; $R^3$ Wasserstoff, Methyl oder Halogen ist und $R^4$ abwesend ist, wenn eine 6(7)- oder 7(8)-Doppelbindung vorliegt ; die 6(7)- und 11(12)-Bindung gesättigt ist, wenn $R^6$ $C_{1-7}$-Alkanoylthio ist ; und $R^1$ abwesend und $R^2$ $C_{1-5}$-Alkyl ist, wenn eine 9(10)-Doppelbindung vorliegt, die Ringe C und D gesättigt sind, n = 0 ist und Z eine Gruppe —COA ist, wobei Verbindungen der Formel I, in denen Z eine Carboxylgruppe ist, auch als Salze vorliegen können.

Eine bevorzugte $C_{1-7}$-Alkanoylgruppe ist Acetyl. Der Ausdruck $C_{1-8}$-Alkyl soll geradkettige oder verzweigte aliphatische oder cycloaliphatische Kohlenwasserstoffreste mit bis zu 8 C-Atomen umfassen. Beispiele solcher Alkylgruppen sind Methyl, Aethyl, Propyl, Butyl, Cyclopentyl, Cyclohexyl.

Als Salze kommen insbesondere Alkalimetallsalze, z. B. Na- und K-, sowie $NH_4$-Salze, ferner Erdalkalimetallsalze, wie Ca-Salze in Betracht. Bevorzugt sind die K-Salze.

Eine bevorzugte Gruppe von Verbindungen der Formel I sind die der Formel

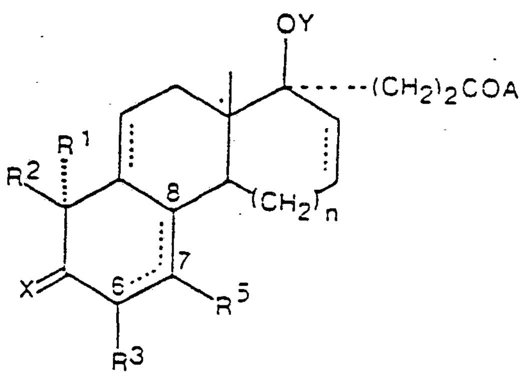

(I-1)

worin $R^1$, $R^2$, $R^3$, $R^5$, A, Y, n und die punktierten Bindungen die oben angegebene Bedeutung haben.

DeA-Steroide der Formel I oder I-1, in denen die Ringe B und C ungesättigt sind, sind neu und als solche ebenfalls Gegenstand der Erfindung.

Eine weitere bevorzugte Gruppe von Verbindungen der Formeln I sind diejenigen, in denen Z Hydroxymethyl ist.

Bevorzugt sind auch Verbindungen der Formeln I und I-1, in denen mindestens ein Rest $R^1$ und $R^2$ $C_{1-5}$ Alkyl ist.

In deA-Steroiden der Formel I, in denen die Kohlenstoffatome in den Stellungen 6 und 7 keine Doppelbindung angehören, können dort befindliche Substituenten $R^3$, $R^4$ und $R^5$ α- oder β-Konfiguration aufweisen, wobei 6α- und 7α-substituierte Verbindungen bevorzugt sind.

Die neuen deA-Steroide der Formel

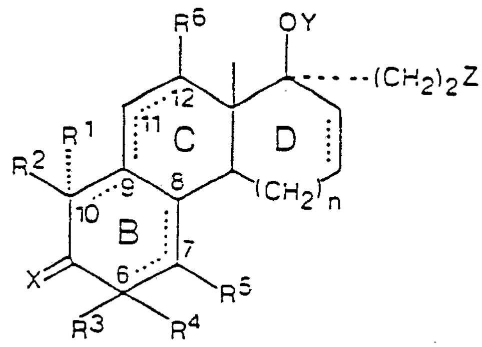

(IA)

in der $R^1$, $R^2$, $R^3$, $R^4$, $R^5$, $R^6$, Y, Z, n und die punktierten Bindungen die den in der Formel I angegebene Bedeutung haben und in den die Ringe B und C ungesättigt sind, können erfindungsgemäss dadurch erhalten werden, dass man

a) in einer Verbindung der Formel

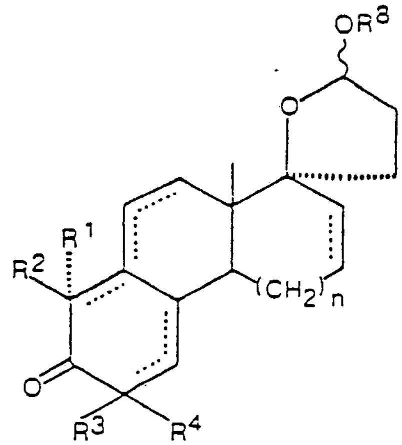

(II)

die Gruppe $OR^8$ zur Oxogruppe oxydiert ; oder

b) eine Verbindung der Formel

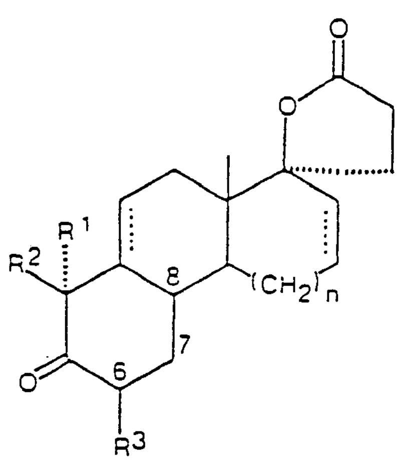

(Ia)

oder die entsprechende Säure oder ein Salz davon in 6(7)- oder 7(8)-Stellung dehydriert ; oder

c) eine Verbindung der Formel

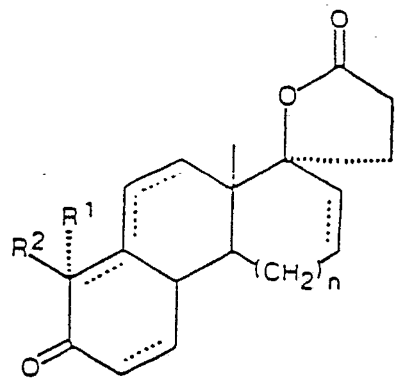

(Ib)

oder die entsprechende Säure oder ein Salz davon halogeniert ; oder

d) eine Verbindung der Formel

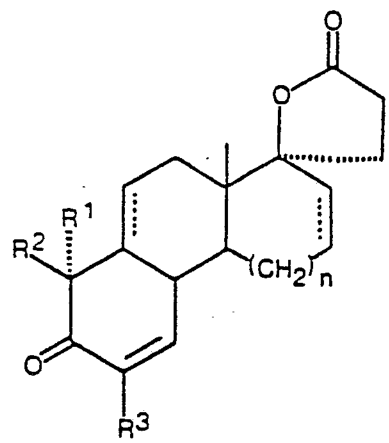

(Ic)

oder

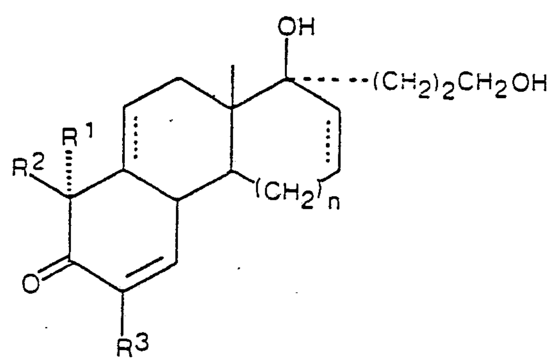

(Id)

mit einer Verbindung $R^7SH$, einem $C_{1-5}$ Alkanol, oder Cyanwasserstoff umsetzt ; oder

e) eine Verbindung der Formel

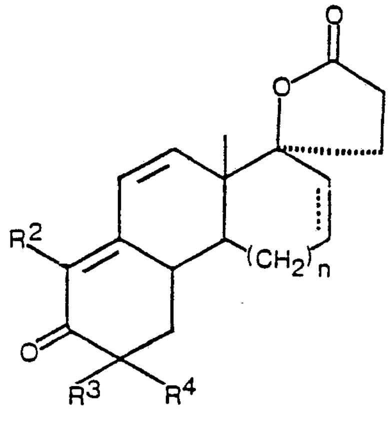

(Ie)

oder

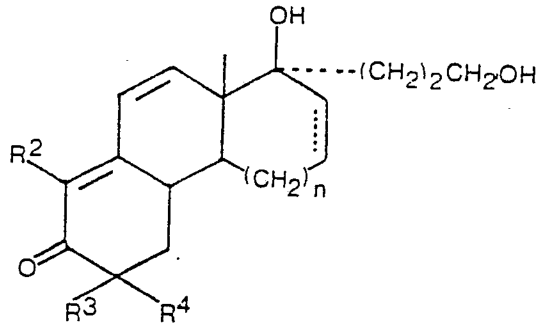

(If)

mit einer Verbindung $R^6SH$ umsetzt ; oder

f) in einer Verbindung der Formel

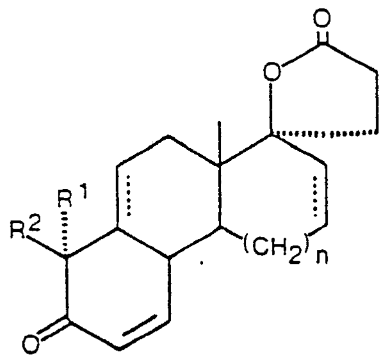

(Ig)

oder an die entsprechende Säure oder deren Salze eine Methylengruppe an die 6(7)-Doppelbindung anlagert ; oder

g) eine Verbindung der Formel

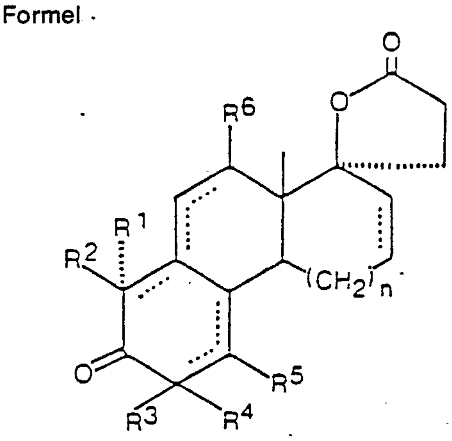

(Ih)

die entsprechende Säure oder ein Salz davon mit einer Methylen-phosphoniumverbindung nach Wittig umsetzt ; oder

h) in einer Verbindung der Formel

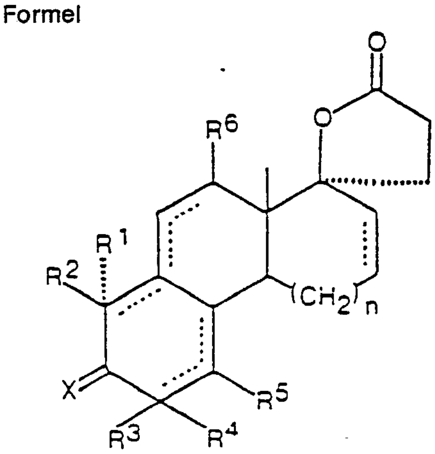

(Ii)

den Lactonring öffnet, oder

i) in einer Verbindung der Formel I, in der Z die Gruppe COA darstellt, diese Gruppe zur Hydroxylmethylgruppe reduziert ;
wobei in den obigen Formeln $R^1$, $R^2$, $R^3$, $R^4$, X, n und die punktierten ringständigen Bindungen die oben angegebene Bedeutung haben und $R^8$ Wasserstoff oder Alkyl darstellt.

Die Oxidation gemäss Verfahrensvariante a) kann mit $CR^{VI}$-Reagentien, insbesondere $CrO_3/H_2SO_4$ (Jones'Reagens) vorgenommen werden. Die Oxidation wird im allgemeinen bei Temperaturen bis zu Raumtemperatur durchgeführt. Bei der Oxidation von Verbindungen mit A = Alkyl, wie z. B. Methyl, kann es zweckmässig sein, bei erhöhter Temperatur, z. B. bei 50 °C zu arbeiten.

Die Dehydrierung gemäss Verfahrensvariante b) wird vorzugsweise in an sich bekannter Weise durch Halogenierung, insbesondere Bromierung, gefolgt von einer Dehydrohalogenierung (Dehydrobromierung) durchgeführt. Die Bromierung kann mit Bromierungsmitteln wie Brom oder Pyridiniumhydrobromid-perbromid erfolgen ; die Dehydrohalogenierung mit Basen wie Collidin, Pyridin oder Lithium-Bromid/Lithiumcarbonat/Dimethylformamid. Die Dehydrierung kann auch direkt, z. B. mit Oxidationsmitteln wie Dichlordicyanobenzochinon oder Chloranil vorgenommen werden.

Die so durchgeführte Dehydrierung liefert ein 6(7)-ungesättigtes deA-Steroid der Formel I. Ein 7(8)-ungesättigtes deA-Steroid der Formel kann daraus durch Dekonjugation der 6(7)-Doppelbindung, z. B. durch Ueberführung der 6(7)-ungesättigten Verbindung in einen 3-Enoläther, z. B. durch Behandlung mit Orthoameisensäureester/p-Toluolsulfosäure und vorsichtige Verseifung der letzteren, z. B. mit schwachen Säuren, wie Ameisensäure oder Essigsäure bei tiefen Temperaturen, z. B. bei 0 °C, erhalten werden.

Die Halogenierung einer Verbindung der Formel Ib (Verfahrensvariante c) kann in an sich bekannter Weise durchgeführt werden, z. B. durch Umsetzung mit einem Halogenierungsmittel, wie einem N-Chloramid oder -imid (z. B. N-Chlorsuccinimid) oder mit elementarem Chlor [vgl. J. Am. Chem, *72*, 4534 (1950)], oder dadurch, dass man eine Verbindung der Formel Ib in einen 3-Enolester oder 3-Enoläther, z. B. das 3-Enolacetat, überführt und danach mit Chlor [vgl. J. Am. Chem. Soc. *82*, 1230 (1960)] ; mit einem N-Chlorimid [vgl. J. Am. Chem. Soc. *82*, 1230 (1960)] ; 77, 3827 (1955) oder Perchlorylfluorid [vgl. J. Am. Chem. Soc. *81*, 5259 (1959) ; Chem. and Ind. *1959*, 1317] umsetzt. Als Fluorierungsmittel kommt weiterhin Trifluormethylhypofluorit in Betracht.

Die Einführung eines Substituenten $R^5$ in eine Verbindung der Formel Ic oder Id (Verfahrensvariante d) kann in an sich bekannter Weise durch Behandlung mit einer Verbindung $R^5SH$, wie einem Mercaptan, z. B. Methyl- oder Aethylmercaptan oder einer Thiocarbonsäure, z. B. Thioessigsäure, bzw. mit einem entsprechenden Alkohol, z. B. Methanol oder Benzylalkohol, erfolgen. Die Reaktion kann in einem inerten Lösungsmittel wie einem Aether, z. B. Dioxan oder Tetrahydrofuran, oder einem Alkohol, wie Methanol oder Aethanol, oder in einem Chlorkohlenwasserstoff, wie Chloroform durchgeführt werden ; das Reagens, z. B. die Thiocarbonsäure wied zweckmässigerweise im Ueberschuss eingesetzt und kann dabei als Lösungsmittel dienen.

Zur Einführung der Cyanogruppe erzeugt man den Cyanwasserstoff zweckmässig in situ, z. B. aus einem Cyanhydrin, wie Acetoncyanhydrin.

Die Einführung des Substituenten $R^6$ in eine Verbindung der Formeln Ie und If (Verfahrensvariante e) kann in Analogie Verfahrensvariante d) durch Behandlung der Ausgangsstoffe mit einer Thiocarbonsäure, z. B. Thioessigsäure, erfolgen.

Die Methylenierung gemäss Verfahrensvariante f) kann in an sich bekannter Weise durchgeführt werden, z. B. mittels Trimethylsulfoxoniumjodid in Gegenwart einer Base, wie Natriumhydrid oder Kalium-t-butylat, in einem aprotischen dipolaren Lösungsmittel, wie Dimethylsulfoxyd, Tetrahydrofuran, Hexamethylphosphorsäuretriamid, Dimethylformamid oder Gemischen hiervon, bei einer Temperatur zwischen etwa 0 und 50 °C, zweckmässig bei Zimmertemperatur.

Die Wittig-Reaktion gemäss Verfahrensvariante g) wird in an sich bekannter Weise in Gegenwart einer starken Base, wie z. B. Buttyllithium, Natriumhydrid, k-tert.-Butylat oder dem Natriumsalz von Dimethylsulfoxid in einem Lösungsmittel, z. B. einem Aether wie Tetrahydrofuran durchgeführt.

Die Aufspaltung des Lactonrings gemäss Verfahrensvariante h) kann in an sich bekannter Weise durchgeführt werden, z. B. mittels einer Base, wie Kalium- oder Natriumhydroxyd, in einem Lösungsmittel, z. B. einem gegebenenfalls wässrigen Alkohol wie (wässriges) Methanol, Aethanol oder Isopropanol, bei einer Temperatur zwischen etwa 0 °C und Rückflusstemperatur des Reaktionsgemisches, zweckmässig bei etwa 50 °C. Die so erhaltenen, der verwendeten Base entsprechenden Salze können durch Ansäuern, z. B. mittels Chlorwasserstoffsäure, in die freien Säuren übergeführt werden. Letztere können durch Umsetzung mit geeigneten Basen in Salze übergeführt werden.

Die Reduktion gemäss Verfahrensvariante i) kann in für die Reduktion von Laktonen zu Alkoholen bekannter Weise, z. B. mit komplexen Metallhydriden wie $LiAlH_4$ vorgenommen werden.

Die Ausgangsverbindungen der Formel II können wie folgt hergestellt werden : eine Verbindung der Formel

(Siehe die Figur, Seite 7 f.)

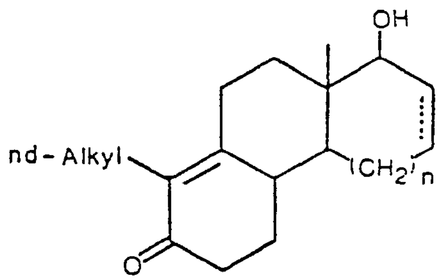

(III)

z. B. Methylcycloketol (nd-Alkyl = Methyl, n = 0, Doppelbindung abwesend) wird mit einem nieder-Alkyljodid z. B. Methyljodid reduktiv alkyliert (J.A.C.S. *89*, 54-64 (1967)). Danach wird die Oxogruppe ketalisiert, die Hydroxygruppe zum 17-Keton oxydiert und mit der Lithiumverbindung von 3-Brompropionaldehyd-dimethylacetal umgesetzt. Danach wird die Dimethylacetalgruppe und die ringständige Ketalgruppe hydrolysiert. Man erhält so eine Verbindung der Formel II, in der $R^1$ und $R^2$ nieder-Alkyl darstellen und in der die 9(11)-Stellung (Steroidnomenklatur) gesättigt ist. Entsprechende 9(11)-ungesättigte Verbindungen der Formel II können wie folgt hergestellt werden : Eine Verbindung der Formel III wird in den tert.Butyläther übergeführt und dieser mit einem nieder-Alkyljodid, z. B. Methyljodid in tert.Butanol in Gegenwart von Kalium-tert.-butylat behandelt, wobei Verbindungen der Formel IV und V erhalten werden.

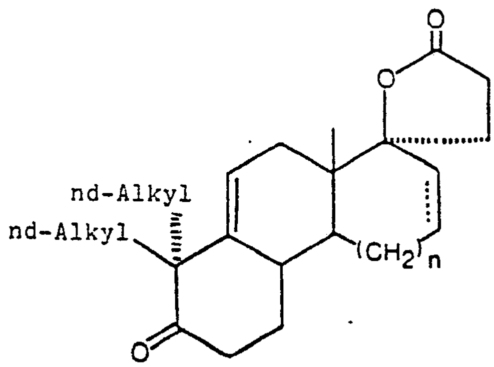

(IV)

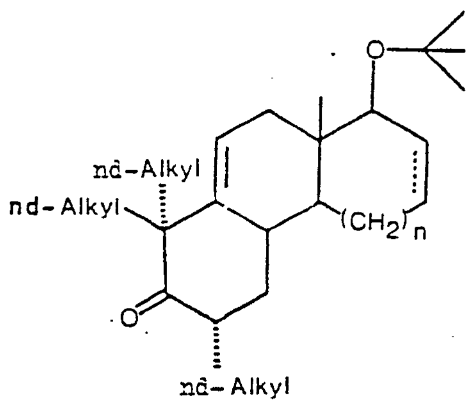

(V)

Nach Spaltung des tert.Butyläthers wird die ringständige Oxogruppe ketalisiert und die Spirolactongruppierung in der oben beschriebenen Weise aufgebaut. Verbindungen der Formel II mit $R^1$ und $R^2$ = Wasserstoff können aus Verbindungen der Formel

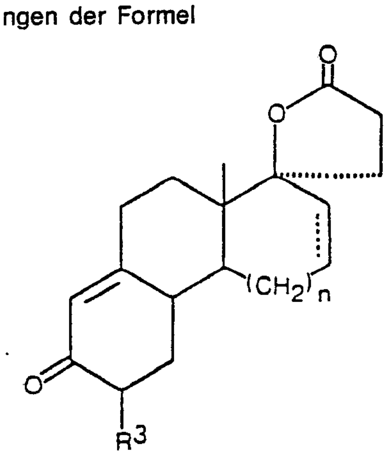

(VI)

durch Hydrierung der konjugierten Doppelbindung, z. B. mit $Pd/H_2$ und gegebenenfalls nachfolgenden 6(7)-Dehydrierung, die in Analogie zu Verfahrensvariante b) ausgeführt werden kann, erhalten werden.

Verbindungen der Formel II, in denen $R^3$ und $R^4$ Methyl darstellen, können aus Verbindungen der Formeln IV, V oder VI durch Methylierung mit Methyljodid in Gegenwart von Lithiumdiisopropylamid in an sich bekannter Weise hergestellt werden.

Eine Doppelbindung in 11(12)-Stellung kann in entsprechende 11(12)-gesättigte Ausgangsstoffe, z. B. Verbindungen der Formel II mit gesättigtem C-Ring, durch Behandlung mit Dehydrierungsmitteln, wie Chloranil, in an sich bekannter Weise eingeführt werden.

Im übrigen können die Ausgangsstoffe wie in den Beispielen beschrieben oder in Analogie dazu hergestellt werden.

Die Verbindungen der Formel I und die Salze hiervon können als Pharmazeutika Verwendung finden. Unter anderem sind sie diuretisch wirksam und geeignet, die Wirkung von Aldosteron oder von Desoxycorticosteron-acetat zu blockieren und können somit beispielsweise als kaliumsparende Diuretika oder zur Ausschwemmung von Oedemen Anwendung finden.

Aus US-A-2 971 009 und J. Org. Chem. 26 (1961), 3910-3915 sind deA-Steroide mit einer 17-Spirolactongruppierung bekannt, die eine hemmende Wirkung auf die Na-retinierende Aktivität von Mineralocorticoiden besitzen. Aus US-A-3 412 107 sind deA-Steroide mit einer 17-Spirolactongruppierung bekannt, die Zwischenprodukte in einer Steroidsynthese sind.

Die Aldosteron-antagonistische Wirkung wurde mit der nachstehend beschriebenen Versuchsanordnung ermittelt : Weibliche Holtzman-Ratten (150-180 g) wurden 70-74 Stunden vor dem Versuch bilateral adrenalektomiert. Nach der Operation erhielten die Tiere 0,9 %ige NaCl-Lösung als Trinkwasser und handelsübliches Trockenfutter. Das Futter wurde 16-17 Stunden vor dem Versuch abgesetzt, während die Kochsalzlösung weiter ad libitum verfügbar blieb. Natrium und Kalium im Harn wurden flammenphotometrisch bestimmt. Die Versuchssubstanzen wurden in 0,3 %iger NaCl-Lösung gelöst und in einer Menge von 30 ml/kg per Magensonde verabreicht. In jedem Versuch wurde eine Kontrollgruppe mitgeführt, die gleich behandelt wurde, jedoch anstelle der Versuchssubstanzen das gleiche Volumen reiner NaCl-Lösung erhielt. 30 Minuten später wurde Aldosteren subcutan injiziert. 120 Minuten nach Verabreichung der Versuchssubstanzen wurden die Harnblasen der Tiere durch leichten suprapubischen Druck entleert. Danach wurden die Tiere in Stoffwechselkäfige ohne Zugang zu Futter und Wasser gesetzt. Der Harn wurde 3-stündlich gesammelt und die Harnblasen wurden am Ende ausgedrückt. Der Spontanharn und der Restharn wurden gesammelt. Die Resultate sind in der nachstehenden Tabelle aufgeführt (Harnvolumen V, $Na^+$ und $K^+$ in % der Kontrollgruppe).

| Substanz | Dosis [mg/kg] | V | $Na^+$ | $K^+$ |
|---|---|---|---|---|
| A | 10 | 119 | 220 | 99 |
| B | 10 | 118 | 217 | 86 |
| | 0,1 | 118 | 122 | 127 |
| C | 10 | 163 | 289 | 101 |
| | 3 | 125 | 202 | 108 |
| | 1 | 118 | 204 | 118 |
| D | 10 | 144 | 246 | 118 |
| | 1 | 179 | 244 | 127 |
| E | 10 | 136 | 284 | 89 |
| | 1 | 171 | 415 | 118 |
| F | 10 | 172 | 200 | 114 |
| | 3 | 132 | 219 | 100 |
| | 1 | 122 | 274 | 103 |
| G | 10 | 93 | 224 | 96 |
| | 1 | 126 | 244 | 99 |
| H | 0,1 | 135 | 213 | 76 |
| I | 10 | 108 | 158 | 85 |
| | 1 | 158 | 293 | 121 |
| J | 10 | 154 | 272 | 107 |
| K | 10 | 153 | 226 | 98 |
| | 1 | 136 | 171 | 114 |

(Fortsetzung)

| Substanz | Dosis [mg/kg] | V | $Na^+$ | $K^+$ |
|---|---|---|---|---|
| L | 10 | 114 | 289 | 84 |
| | 1 | 113 | 220 | 116 |
| M | 10 | 113 | 254 | 55 |
| | 1 | 106 | 245 | 108 |
| N | 1 | 114 | 224 | 75 |
| | 0,1 | 158 | 297 | 86 |

A : 5-Oxo-A-tetranor-10α,17a-pregn-6-en-21,17-carbolacton
B : 7α-(Acetylthio)-5-oxo-A-tetranor-10α,17a-pregnan-21,17-carbolacton
C : Kalium-17-hydroxy-5-oxo-A-tetranor-10α,17a-pregn-6-en-21-carboxylat
D : 5-Oxo-1,5-seco-A-trinor-17α-pregnan-21,17-carbolacton
E : 7α-(Acetylthio)-5-oxo-1,5-seco-A-trinor-17α-pregnan-21,17-carbolacton
F : Kalium-17-hydroxy-5-oxo-1,5-seco-A-trinor-17α-pregn-6-en-21-carboxylat
G : 6-Brom-5-oxol-1,5-seco-A-trinor-17α-pregn-6-en-21,17-carbolacton
H . 5-Oxo-A-tetranor-17α-pregna-9,11-dien-21,17-carbolacton
I : 7α-(Aethylthio)-5-oxo-1,5-seco-A-trinor-17α-pregnan-21,17-carbolacton
J : 7α-Cyano-5-oxo-1,5-seco-A-trinor-17α-pregnan-21,17-carbolacton
K : Kalium-17-hydroxy-5-oxo-1,5-seco-A-trinor-17α-pregna-6,9(11)-dien-21-carboxylat
I : 6α-Methyl-5-oxo-1,5-seco-A-trinor-17α-pregn-9(11)en-21,17-carbolacton
M : 7α-(Acetylthio)-5-oxo-3,5-seco-A-nor-10α,17a-pregnan-21,17-carbolacton
N : 6,6-Dimethyl-5-oxo-1,5-seco-A-trinor-17α-pregn-9(11)-en-21.17-carbolacton

Die Verbindungen der Formel I und ihre Salze können als Heilmittel z. B. in Form pharmazeutischer Präparate Verwendung finden, welche sie oder ihre Salze in Mischung mit einem für die enterale oder parenterale Applikation geeigneten organischen oder anorganischen inerten Trägermaterial, wie z. B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z. B. als Tabletten, Dragées, Suppositorien, Kapseln ; oder in flüssiger Form, z. B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes oder Puffer. Sie können auch noch andere therapeutisch wertvolle Stoffe enthalten.

Die Herstellung der Arzneimittel kann in an sich bekannter Weise erfolgen, indem man die Verbindung der Formel I bzw. ein Salz hiervon mit zur therapeutischen Verabreichung geeigneten, nichttoxischen, inerten, an sich in solchen Präparaten üblichen festen und/oder flüssigen Trägermaterialien, wie z. B. den vorstehend genannten, vermischt und gegebenenfalls in die gewünschte Form bringt. Als Dosiseinheit zur Verabreichung an den Erwachsenen kommen etwa 0,1-10 mg/kg pro Tag in Betracht.

## Beispiel 1

Zu einer Lösung von 9,6 g 5-Oxo-1,5-seco-A-trinor-17α-pregna-21,17-carbolacton in 100 ml Tetrahydrofuran wird innerhalb 20 Minuten eine Lösung von 22,9 g 2-Carboxy-äthyl-triphenylphosphoniumperbromid in 125 ml Tetrahydrofuran getropft. Nach 2 Stunden Rühren bei Raumtemperatur wird das Reaktionsgemisch auf Eiswasser gegossen und mit Methylenchlorid extrahiert. Das durch Aufarbeiten des Methylenchlorid-Extraktes erhaltene Rohprodukt wird 1 Stunde unter Argon mit einem Gemisch von 105 ml Dimethylformamid, 10,5 g Lithiumbromid und 10,5 g Lithiumcarbonat gekocht. Die erkaltete Lösung wird auf Wasser gegossen und mit einem Gemisch von Aether-Methylenchlorid (4 : 1) extrahiert. Durch Chromatographie des aus dem Extrakt erhaltenen Rückstandes an Kieselgel mit Toluol-Aethylacetat (7 : 3) erhält man als Hauptprodukt 5-Oxo-1,5-seco-A-trinor-17α-pregn-6-en-21,17-carbolacton, Schmelzpunkt 128-129° (aus Diäthyläther) ; $[\alpha]_D^{25}$ – 112,5° (Dioxan, c = 0,2).

Als Nebenprodukt erhält man 6-Brom-5-oxo-1,5-seco-A-trinor-17α-pregn-6-en-21,17-carbolacton, Schmelzpunkt 194-195° (aus Diäthyläther-Methylenchlorid) ; $[\alpha]_D^{25}$ – 38° (Dioxan, c = 0,2)

## Beispiel 2

In Analogie zu Beispiel 1 erhält man aus 5-Oxo-1,5-seco-A-trinor-17α-pregn-9(11)-en-21,17-carbolacton das 5-Oxo-1,5-seco-A-trinor-17α-pregna-6,9(11)-dien-21,17-carbolacton, Schmelzpunkt 195-197° ; $[\alpha]_D^{25}$ – 292° (Dioxan, c = 0,5).

Als Nebenprodukte kann man 5-Oxo-1,5-seco-A-trinor-17α-pregna-7,9(11)-dien-21,17-carbolacton, Schmelzpunkt 159-160° ; $[\alpha]_D^{25}$ + 40,5 (Dioxan, c = 0,2) isolieren.

Beispiel 3

In Analogie zu Beispiel 1 erhält man aus 5-Oxo-3,5-seco-A-nor-17α-pregna-21,17-carbolacton das 5-Oxo-3,5-seco-A-nor-17α-pregn-6-en-21,17-carbolacton, Schmelzpunkt 181-183° ; $[\alpha]_D^{25}$ − 92,5° (Dioxan, c = 1,0).

Als Nebenprodukt erhält man 6-Brom-5-oxo-3,5-seco-A-nor-17α-pregn-6-en-21,17-carbolacton, Schmelzpunkt 194-196° ; $[\alpha]_D^{25}$ − 40,1° (Dioxan, c = 1,0).

Beispiel 4

In Analogie zu Beispiel 1 erhält man aus 5-Oxo-2,5-seco-A-dinor-17α-pregnan-21,17-carbolacton das 5-Oxo-2,5-seco-A-dinor-17α-pregn-6-en-21,17-carbolacton, Schmelzpunkt 157-159° ; $[a]_D^{25}$ − 101,3° (Dioxan, c = 1,0).

Als Nebenprodukt erhält man 6-Brom-5-oxo-2,5-seco-A-dinor-17α-pregn-6-en-21,17-carbolacton, Schmelzpunkt 194-196° ; $[\alpha]_D^{25}$ − 48° (Dioxan, c = 1,0).

Beispiel 5

In Analogie zu Beispiel 1 erhält man aus 6-Methyl-5-oxo-1,5-seco-A-trinor-17α-pregnan-21,17-carbolacton das 6-Methyl-5-oxo-1,5-seco-A-trinor-17α-pregn-6-en-21,17-carbolacton, Schmelzpunkt 181-182° ; $[\alpha]_D^{25}$ − 86° (Dioxan, c = 0,5).

Beispiel 6

In Analogie zu Beispiel 1 erhält man aus 6-Methyl-5-oxo-1,5-seco-A-trinor-17α-pregn-9(11)-en-21,17-carbolacton das 6-Methyl-5-oxo-1,5-seco-A-trinor-17α-pregna-6,9(11)-dien-21,7-carbolacton, Schmelzpunkt 195-198° ; $[\alpha]_D^{25}$ − 256,4° (Dioxan, c = 0,5).

Beispiel 7

In Analogie zu Beispiel 1 erhält man aus 5-Oxo-3,5-seco-A-nor-10α, 17α-pregnan-21,17-carbolacton das 5-Oxo-3,5-seco-A-nor-10α,17α-pregn-6-en-21,17-carbolacton, Schmelzpunkt 149-150° ; $[\alpha]_D^{25}$ − 119,2° (Dioxan, c = 0,5).

Als Nebenprodukt erhält man 6-Brom-5-oxo-3,5-seco-A-nor-10α,17α-pregn-6-en-21,17-carbolacton, Schmelzpunkt 179-180°.

Das Ausgangsmaterial wird wie folgt hergestellt :

Methylcycloacetal wird mit n-Propyljodid reduktiv alkyliert. Man erhält 17β-Hydroxy-3,5-seco-A-nor-10α-androstan-5-on, Schmelzpunkt 117-118,5° und daraus durch Ketalisierung das 17β-Hydroxy-3,5-seco-A-nor-10α-androstan-5-on-äthylen-acetal, Schmelzpunkt 153-154°.

Oxydation mit Pyridiniumchlorchromat liefert 3,5-Seco-A-nor-10α-androstan-5,17-dion-5-(äthylenacetal), $[\alpha]_D$ − 108° (Dioxan 0,36) aus dem in Analogie zu Beispiel 41 das 5-Oxo-3,5-seco-A-nor-10α,17α-pregnan-21,17-carbolacton, Schmelzpunkt 150-151° erhalten wird.

Beispiel 8

6 g 5-oxo-A-tetranor-10α-17α-pregnan-21,17-carbolacton werden in 900 ml Aether gelöst und im Eisbad auf ca. 3° gekühlt. Die Lösung wird unter Rühren innerhalb 10 Minuten mit einer Lösung von 3 368 mg Brom in 12,42 ml Essigsäure tropfenweise versetzt. Zu der praktisch farblosen Lösung wird Natriumsulfitlösung gegeben und anschliessend mit Diäthyläther extrahiert. Nach Eindampfen des Extraktes erhält man 8,62 g farbloses Oel, das mit einer Suspension von 6 g Lithiumcarbonat und 6 g Lithiumbromid 1 Stunde unter Argon gekocht wird. Nach Aufarbeitung des Reaktionsgemisches und Chromatographie des Rückstandes an Kieselgel mit Toluol-Aethylacetat (9 : 1) erhält man 5-Oxo-A-tetranor-10α,17α-pregn-6-en-21,17-carbolacton.

Beispiel 9

Zu einer auf 15° gekühlten Lösung von 10 g 5-Oxo-1,5-seco-A-trinor-17α-pregn-6-en-21,17-carbolacton in 330 ml Pyridin werden innerhalb 5 Minuten 5,6 ml Sulfurylchlorid gegeben. Die Reaktionslösung wird 1 Stunde bei 15° gehalten, dann auf Eiswasser gegossen und mit Aether extrahiert. Der ätherische Extrakt wird mit verdünnter Salzsäure und Wasser gewaschen, über Natriumsulfat getrocknet und unter

vermindertem Druck eingedampft. Der Rückstand wird an 800 g Silicagel chromatographiert. Mit Toluol-Aethylacetat (95 : 5) werden 1,8 g reines 6-Chlor-5-oxo-1,5-seco-A-trinor-17α-pregn-6-en-21,17-carbolacton, Schmelzpunkt 220-223° (aus Aceton-Hexan) ; $[\alpha]_D^{25}$ − 57,2° (Dioxan, c = 1,0) eluiert.

Beispiel 10

658 mg 6-Brom-5-oxo-1,5-seco-A-trinor-17α-pregn-6-en-21,7-carbolacton werden in einem Gemisch von 10 ml Methanol und 2,15 ml Wasser suspendiert. Zu dieser Suspension werden unter Argon 2,15 ml Thioessigsäure gegeben. Nach 3 Studen wird das Reaktionsgemisch mit Wasser verdünnt. Der Rückstand wird abgenutscht, getrocknet und zweimal aus Aceton kristallisiert. Man erhält 7α-(Acetylthio)-6α-brom-5-oxo-1,5-seco-A-trinor-17α-pregnan-21,17-carbolacton, Schmelzpunkt 178-181° (Zersetzung) ; $[\alpha]_D^{25}$ − 18,4° (Dioxan, c = 0,5).

Beispiel 11

520 mg 6-Brom-5-oxo-1,5-seco-A-trinor-17α-pregn-6-en-21,17-carbolacton werden in 15 ml Isopropanol gelöst. Die Lösung wird mit 0,68 ml 2N Kalilauge versetzt und das Reaktionsgemisch unter Argonatmosphäre 2 Stunden stehen gelassen. Nach Eindampfen des Reaktionsgemisches erhält man einen stark hygroskopischen Schaum von Kalium 6-Brom-17-hydroxy-5-oxo-1,5-seco-A-trinor-17α-pregn-6-en-21-carboxylat, $[\alpha]_D^{25}$ − 55,5° (Aethanol, c = 0,2).

Beispiel 12

In Analogie zu Beispiel 11 erhält man aus 6-Chlor-5-oxo-1,5-seco-A-trinor-17α-pregn-6-en-21,17-carbolacton das Kalium 6-Chlor-17-hydroxy-5-oxo-1,5-seco-A-trinor-17α-pregn-6-en-21-carboxylat als amorphe Substanz, $[\alpha]_D^{25}$ − 67,1° (Methanol, c = 1,0).

Beispiel 13

4,4 g Trimethylsulfoxoniumjodid und 0,82 g Natriumhydrid-Dispersion (50 %ig in Mineralöl) werden unter Argonatmosphäre bei ca. 15° mit 15 ml Dimethylsulfoxid versetzt. Nach 2 Stunden Rühren bei Zimmertemperatur wird das Gemisch mit einer Lösung von 3,02 g 5-Oxo-1,5-seco-A-trinor-17α-pregn-6-en-21,17-carbolacton in 15 ml Dimethylsulfoxid versetzt. Das Reaktionsgemisch wird über Nacht bei Zimmertemperatur unter Argon gerührt und danach tropfenweise bei 10° mit 1 ml Eisessig versetzt. Das Reaktionsgemisch wird dann in Wasser gegossen, mit Aether extrahiert, der Extrakt gewaschen, getrocknet und eingedampft. Der Rückstand wird an Kieselgel mit Hexan-Aether (2 : 1) chromatographiert und liefert nach Kristallisation aus Methylenchlorid-Diäthyläther-Hexan reines 6,7-Dihydro-5-oxo-3'H-cyclopropa [6,7]-1,5-seco-A-trinor-17α-pregn-6-en-21,17-carbolacton in zwei diastereomeren Formen. Ein Isomer (vermutlich die 6β,7β-Dihydro-Form) zeigt einen Schmelzpunkt 214-216° ; $[\alpha]_D^{25}$ − 105° (Dioxan, c = 0,5). Das andere Isomer (vermutlich die 6α,7α-Dihydro-Form) schmilzt bei 178-179°, $[\alpha]_D^{25}$ − 113° (Dioxan, c = 0,2).

Beispiel 14

Zu einer Lösung von 3,5 g 5-Oxo-1,5-seco-A-trinor-17α-pregn-6-en-21,17-carbolacton in 123 ml Isopropanol werden 5,64 ml 2N Kalilauge gegeben. Das Reaktionsgemisch wird 2 Stunden unter Argon bei Zimmertemperatur gerührt. Zur Aufarbeitung wird ein Teil des Isopropanols (etwa 70 %) abdestilliert, worauf nach dem Abkühlen das Kalium-17-Hydroxy-5-oxo-1,5-seco-A-trinor-17α-pregn-6-en-21-carboxylat in Form farbloser hygroskopischer Kristalle auskristallisiert. Schmelzpunkt 240-242° (Zersetzung) ; $[\alpha]_D^{25}$ − 120,5° (Aethanol, c = 0,2).

Beispiel 15

In Analogie zu Beispiel 14 erhält man aus 5-Oxo-1,5-seco-A-trinor-17α-pregna-6,9(11)-dien-21,17-carbolacton das Kalium-17-hydroxy-5-oxo-1,5-seco-A-trinor-17α-pregna-6,9(11)-dien-21-carboxylat, Schmelzpunkt ab 110° (beginnende Zersetzung) ; $[\alpha]_D^{25}$ − 242° (Aethanol, c = 0,2).

Beispiel 16

In Analogie zu Beispiel 14 erhält man aus 6-Methyl-5-oxo-1,5-seco-A-trinor-17α-pregn-6-en-21,17-carbolacton das Kalium-17-hydroxy-6-methyl-5-oxo-1,5-seco-A-trinor-17α-pregn-6-en-21-carboxylat als stark hygroskopischen Schaum, Schmelzpunkt 265-268° (Zersetzung) ; $[\alpha]_D^{25}$ − 96° (Aethanol, c = 0,5).

Beispiel 17

In Analogie zu Beispiel 14 erhält man aus 6-Methyl-5-oxo-1,5-seco-A-trinor-17α-pregna-6,9(11)-dien)21,17-carbolacton das Kalium-17-hydroxy-6-methyl-5-oxo-1,5-seco-A-trinor-17α-pregna-6,9(11)-dien-21-carboxylat, Schmelzpunkt 160-165° ; $[\alpha]_D^{25}$ – 230,5° (Aethanol, c = 0,2).

Beispiel 18

In Analogie zu Beispiel 14 erhält man aus 5-Oxo-3,5-seco-A-nor-10-17-pregn-6-en-21,17-carbolacton das Kalium-17-hydroxy-5-oxo-3,5-seco-A-nor-10α,17α-pregn-6-en-21-carboxylat, Schmelzpunkt 256-260° ; $[\alpha]_D^{25}$ – 121,4° (Aethanol, c = 0,5).

Beispiel 19

In Analogie zu Beispiel 14 erhält man aus 5-Oxo-A-tetranor-10α,17α-pregn-6-en-21,17-carbolacton das Kalium-17-hydroxy-5-oxo-A-tetranor-10α,17α-pregn-6-en-21-carboxylat, Schmelzpunkt 225-230° (Zersetzung) ; $[\alpha]_D^{25}$ – 102° (Dioxan, c = 0,2).

Beispiel 20

In Analogie zu Beispiel 14 erhält man aus 6,7-Dihydro-5-oxo-3'H-cyclopropa [6,7]-1,5-seco-A-trinor-17α-pregn-6-en-21,17-carbolacton das Kalium-6,7-Dihydro-17-hydroxy-5-oxo-3'H-cyclopropa [6,7]-1,5-seco-A-trinor-17α-pregn-6-en-21-carboxylat, Schmelzpunkt 265-267° ; $[\alpha]_D^{25}$ – 86° (Dioxan, c = 0,5).

Beispiel 21

In Analogie zu Beispiel 14 erhält man aus 5-Oxo-3,5-seco-A-nor-17α-pregn-6-en-21,17-carbolacton das Kalium-17-hydroxy-5-oxo-3,5-seco-A-nor-17α-pregn-6-en-21-carboxylat als amorphe Substanz, $[\alpha]_D^{25}$ – 86,4° (Methanol, c = 1).

Beispiel 22

5 g 5-Oxo-1,5-seco-A-trinor-17α-pregn-6-en-21,17-carbolacton werden in 20,5 ml Methanol und 4,4 ml Wasser suspendiert. Die Suspension wird während 10 Minuten unter Argon tropfenweise mit 4,4 ml Thioessigsäure versetzt. Nach 3-stündigem Rühren bei Raumtemperatur ist auf dem Dünnschichtchromatogramm kein Ausgangsmaterial mehr nachzuweisen. Die Lösung wird dann auf Eiswasser gegossen, der Niederschlag abgenutscht und aus Methanol-Wasser umkristallisiert. Man erhält 7α-(Acetylthio)-5-oxo-1,5-seco-A-trinor-17α-pregnan-2,17-carbolacton vom Schmelzpunkt 147-148° ; $[\alpha]_D^{25}$ – 27° (Dioxan, c = 0,2).

Beispiel 23

In Analogie zu Beispiel 22 erhält man aus 5-Oxo-1,5-seco-A-trinor-17α-pregna-6,9(11)-dien-21,17-carbolacton das 7α-(Acetylthio)-5-oxo-1,5-seco-A-trinor-17α-pregn-9(11)-en-21,17-carbolacton vom Schmelzpunkt 153-154° ; $[\alpha]_D^{25}$ –113° (Dioxan, c = 0,2), sowie das 7β-(Acetylthio)-5-oxo-1,5-seco-A-trinor-17α-pregn-9(11)-en-21,17-carbolacton vom Schmelzpunkt 158-162° ; $[\alpha]_D^{25}$ – 84,5° (Dioxan, c = 0,2).

Beispiel 24

In Analogie zu Beispiel 22 erhält man aus 6-Methyl-5-oxo-1,5-seco-A-trinor-17α-pregn-6-en-21,17-carbolacton nach Chromatographie des Reaktionsproduktes an Kieselgel mit Hexan-Aethylacetat (4 : 1) das 7α-(Acetylthio)-6α-methyl-5-oxo-1,5-seco-A-trinor-17α-pregnan-21,17-carbolacton, Schmelzpunkt 203-205° ; $[\alpha]_D^{25}$ 0° (Dioxan, c = 0,2).

Beispiel 25

In Analogie zu Beispiel 22 erhält man aus 6-Methyl-5-oxo-1,5-seco-A-trinor-17α-pregna-6,9(11)-dien-21,17-carbolacton das 7α-(Acetylthio)-6α-methyl-5-oxo-1,5-seco-A-trinor-17α-pregn-9(11)-en-21,17-carbolacton vom Schmelzpunkt 207-210° ; $[\alpha]_D^{25}$ – 7,5° (Dioxan, c = 0,2).

Beispiel 26

In Analogie zu Beispiel 22 erhält man aus 5-Oxo-3,5-seco-A-nor-10α-17α-pregn-6-en-21,17-carbolacton das 7α-(Acetylthio)-5-oxo-3,5-seco-A-nor-10α, 17α-pregnan-21,17-carbolacton, Schmelzpunkt 168-169° ; $[\alpha]_D^{25}$ – 18° (Dioxan, c = 0,5).

Aus den Mutterlaugen lässt sich das 7β-Isomere isolieren.

Beispiel 27

In Analogie zu Beispiel 22 erhält man aus 5-Oxo-A-tetranor-10α-17α-pregn-6-en-21,17-carbolacton das 7α-(Acetylthio)-5-oxo-A-tetranor-10α,17α-pregnan-21,17-carbolacton.

Beispiel 28

In Analogie zu Beispiel 22 erhält man aus 5-Oxo-3,5-seco-A-nor-17α-pregn-6-en-21,17-carbolacton das 7α-(Acetylthio)-5-oxo-3,5-seco-A-nor-17α-pregnan-21,17-carbolacton, Schmelzpunkt 162-165°, $[\alpha]_D^{25}$ − 17,2° (Dioxan, c = 1).

Beispiel 29

In Analogie zu Beispiel 22 erhält man aus 5-Oxo-2,5-seco-A-dinor-17α-pregn-6-en-21,17-carbolacton das 7α-(Acetylthio)-5-oxo-2,5-seco-A-dinor-17α-pregnan-21,17-carbolacton, Schmelzpunkt 194-196°; $[\alpha]_D^{25}$ − 9° (Dioxan, c = 1).

Beispiel 30

In Analogie zu Beispiel 22 erhält man aus 6-chlor-5-oxo-1,5-seco-A-trinor-17α-pregn-6-en-21,17-carbolacton das 7α-(Acetylthio)-6α-chlor-5-oxo-1,5-seco-A-trinor-17α-pregnan-21,17-carbolacton, Schmelzpunkt 204-209°: $[\alpha]_D^{25}$ − 17,3°.

Beispiel 31

In Analogie zu Beispiel 22 erhält man aus 5-Oxo-1,5-seco-A-trinor-17α-pregn-6-en-21,17-carbolacton mit Thiopropionsäure das 7α-(Propionylthio)-5-oxo-1,5-seco-A-trinor-17α-pregnan-21,17-carbolacton, Schmelzpunkt 148-149°; $[\alpha]_D^{25}$ − 31,1°.

Beispiel 32

Eine Lösung von 3,02 g 5-Oxo-1,5-seco-A-trinor-17α-pregn-6-en-21,17-carbolacton in 20 ml Aethylmercaptan und 2 ml Piperidin wird 90 Minuten bei Raumtemperatur gehalten. Zur Aufarbeitung wird das Lösungsmittel unter vermindertem Druck eingedampft und der Rückstand aus Aceton-Hexan umkristallisiert. Man erhält 2,9 g reines 7α-(Aethylthio)-5-oxo-1,5-seco-A-trinor-17α-pregnan-21,17-carbolacton, Schmelzpunkt 180-181°; $[\alpha]_D^{25}$ − 51,9°.

Beispiel 33

Zu einer Lösung von 2 g 5-Oxo-1,5-seco-A-trinor-17α-pregn-6-en-21,17-carbolacton in 40 ml Methanol und 4 ml Tetrahydrofuran werden 2 ml gesättigte Natriumcarbonatlösung und 1,3 ml Acetoncyanhydrin gegeben. Das Reaktionsgemisch wird 2 Stunden unter Rühren zum Rückfluss erhitzt. Zur Aufarbeitung wird die Lösung abgekühlt, auf Eiswasser gegossen und mit Aether extrahiert. Die ätherischen Extrakte liefern 2,2 g farblose Kristalle die nach Chromatographie auf Silicagel und Kristallisation aus Aceton-Hexan reines 7α-Cyano-5-oxo-1,5-seco-A-trinor-17α-pregnan-21,17-carbolacton geben. Schmelzpunkt 240-241°; $[\alpha]_D^{25}$ − 5,7°.

Beispiel 34

In Analogie zu Beispiel 1 wird 5-Oxo-1,5-seco-A-trinor-17α-pregna-21,17-carbolacton mit 2,2 Mol Aequivalenten Brom umgesetzt. Man erhält nach Aufarbeitung wie in Beispiel 1 als Hauptprodukt das 6-Brom-5-oxo-1,5-seco-A-trinor-17α-pregn-6-en-21,17-carbolacton.

Beispiel 35

1 500 mg 5-Oxo-1,5-seco-A-trinor-17α-pregn-6-en-21,17-carbolacton werden in einem Gemisch von 30 ml Methanol und 4 ml wässriger 2N Natronlauge 7 Tage lang unter Argon zum Rückfluss erhitzt. Das Reaktionsgemisch wird dann mit Wasser verdünnt, mit 2N Salzsäure angesäuert und mit Aether-Methylenchlorid (4 : 1) extrahiert. Die organischen Phasen werden mit Kochsalzlösung gewaschen, über Magnesiumsulfat getrocknet und unter vermindertem Druck eingedampft. Der Rückstand wird an der 100-fachen Menge Kieselgel mit Toluol-Essigester (19 : 1) chromatographiert. Als unpolares Produkt wird zuerst 5-Oxo-1,5-seco-A-trinor-17α-pregn-7-en-21,17-carbolacton isoliert, das nach Kristallisation aus Aceton-Hexan bei 153-157° schmilzt. $[\alpha]_D$ = 8,5° (Dioxan, c = 0,2). Aus den nachfolgenden Fraktionen werden nicht umgesetztes Ausgangsmaterial, 7α-Methoxy-5-oxo-1,5-seco-A-trinor-17α-pregnan-21,17-

carbolacton, Schmelzpunkt 138-142° (aus Aether), $[\alpha]_D$ − 12,5° (Dioxan, c = 0,2) sowie das 7-Methoxy-5-oxo-1,5-seco-A-trinor-17α-pregn-6-en-21,17-carbolacton, Schmelzpunkt 167-169° (aus Methylenchlorid-Aether), $[\alpha]_D$ − 63° (Dioxan, c = 0,2) erhalten.

## Beispiel 36

2 g 5-Oxo-A-tetranor-17α-pregna-9,11-dien-21,17-carbolacton werden in 40 ml Isopropanol gelöst und mit 3,42 ml Kaliumhydroxydlösung 2N versetzt und während 1 Stunde in einer Argonatmosphäre gerührt. Anschliessend wird die Lösung unter mehrmaliger Zugabe von Isopropanol eingeengt (azeotrope Entfernung des Wassers) und unter starkem Rühren mit der 6-fachen Menge Aceton versetzt, worauf das K-Salz ausfällt. Nach dem Abkühlen über Nacht auf + 5° wird abgenutscht und sofort im Hochvakuum bei 80° getrocknet. Man erhält so 1,83 g Kalium-17-hydroxy-5-oxo-A-tetranor-17α-pregna-9,11-dien-21-carboxylat, das bei 169° sintert (anschliessend Schaumbildung). [α] − 113,4 (EtOH 0,5), UV λ = 292 nm, ε = 24 950.

Analog wird das Natrium-17-hydroxy-5-oxo-A-tetranor-17α-pregna-9,11-dien-21-carboxylat hergestellt. $[\alpha]_D$ − 118° (EtOH 0,5 %), UV λ = 293 mm, ε = 22 600.

## Beispiel 37

2,6 g 5-Oxo-A-tetranor-17α-pregn-9-en-21,17-carbolacton werden in 39 ml t-Butanol gelöst und zusammen mit 2,6 g Chloranil während 5 Stunden in einer Argonatmosphäre am Rückfluss gekocht. Das abgekühlte Reaktionsprodukt wird auf Eiswasser gegossen und mit Aether extrahiert. Die organischen Phasen werden zweimal mit 0,1N NaOH, dann mit Kochsalzlösung neutral gewaschen. Das so erhaltene Rohprodukt wird an der 100-fachen Menge Kieselgel mit Hexan-Essigester 2 : 1 chromatographiert. Das Rohprodukt wird aus Aceton-Isopropyläther kristallisiert. Man erhält so das 5-Oxo-A-tetranor-17α-pregna-9,11-dien-21,17-carbolacton, Schmelzpunkt 122-123°. $[\alpha]_D^{25}$ − 211° (Dioxan 0,2), UV λ = 289 nm, ε = 26 200.

Analog erhält man aus 5-Oxo-1,2,3,4,19-pentanor-17α-pregn-9-en-21,17-carbolacton das 5-Oxo-1,2,3,4,19-pentanor-17α-pregna-9,11-dien-21,17-carbolacton. Schmelzpunkt 153-154° (Aus Methylenchlorid/Aether), UV $\lambda_{MAX}$ = 180 nm, ε = 27 000. $[\alpha]_D$ − 234° (Dioxan 0,5 %).

## Beispiel 38

433 mg 5-Oxo-A-tetranor-17α-pregna-9,11-dien-21,17-carbolacton werden in 8,7 ml Isopropanol und 8,7 ml Wasser gelöst. Zur Lösung werden 56,4 mg Calciumhydroxyd zugegeben. Man rührt in einer Argonatmosphäre während 2 Stunden bei 50° und engt dann die Reaktionslösung unter mehrmaliger Zugabe von Isopropanol ein. Nach Zusatz von Essigester wird das Produkt abgenutscht und getrocknet. Man erhält so 386 mg Calcium-17-hydroxy-5-oxo-A-tetranor-17α-pregna-9,11-dien-21-carboxylat.

## Beispiel 39

300 mg Lithiumaluminiumhydrid werden in 37,9 ml Tetrahydrofuran suspendiert. Dazu wird eine Lösung von 1,6 g 5-Oxo-A-tetranor-17α-pregna-9,11-dien-21,17-carbolacton in 46 ml Tetrahydrofuran zugetropft und anschliessend wird während 1 Stunde unter Argon am Rückfluss gekocht. Unter Kühlung werden 2,5 ml Aethylacetat und anschliessend 50 ml Salzsäure 0,5N zugegeben. Nach Aufarbeiten des Reaktionsgemisches erhält man 1,5 g rohes 5,17 β-Dihydroxy-17-(3-hydroxypropyl)-A-tetranorandrosta-9,11-dien.

1,1 g des so erhaltenen Rohproduktes werden zusammen mit 1,38 g Dichlordicyanchinon in 45,8 ml Dioxan suspendiert und unter Argon während 48 Stunden bei Zimmertemperatur gerührt. Zur Aufarbeitung wird auf Eis/Wasser gegossen und mit Aether extrahiert. Die Extrakte werden mit Natriumbicarbonatlösung und anschliessend mit Kochsalzlösung neutral gewaschen. Das so erhaltene Rohprodukt wird an Kieselgel mit Hexan-Toluol 1 : 1 und schliesslich mit Toluol chromatographiert. Kristallisation aus Methylenchlorid/Hexan liefert 400 mg 17β-Hydroxy-17-(3-hydroxypropyl)-A-tetranorandrosta-9,11-dien-5-on als gelbliche Kristalle vom Schmelzpunkt 168-169°. $[\alpha]_D$ − 170° (Dioxan 0,2 %). UV λ = 292 nm, ε = 26 000.

## Beispiel 40

610 mg 5-Oxo-A-tetranor-17α-pregna-9,11-diene-21,17-carbolacton werden in 5 ml Thioessigsäure während 5 Stunden am Rückfluss unter Argon gekocht. Zur Aufarbeitung wird auf Eis/Wasser gegossen und mit Aether extrahiert. Die organischen Phasen werden zuerst mit 1n Kalilauge, dann mit Wasser bis neutral gewaschen. Nach dem Trocknen mit Magnesiumsulfat und Eindampfen wurde das Rohprodukt mit Toluol -Aethylacetat an 150 g Kieselgel chromatographiert. Die zuerst eluierte Fraktion besteht aus nicht umgesetzten Edukt. Aus der zweiten Fraktion wird durch zweimalige Kristallisation aus Aceton-Isopropylalkohol 36 mg reines 12β-(Acetylthio)-5-oxo-A-tetranor-17α-pregn-9-ene-21,17-carbolactone Schmelzpunkt 195-198° erhalten. $[\alpha]_D$ − 45° (Dioxan).

Beispiel 41

10 g 1,5-Seco-A-trinorandrostan-5,17-dien-5,5-(äthylen) acetal werden in 168 ml Tetrahydrofuran gelöst und auf − 20° abgekühlt. Nun werden 2,35 g fein geschnittener Draht zugegeben und anschliessend werden innert 2 Stunden 36,5 g 3-Brompropionaldehyd-dimethylacetal zugetropft. Das Kühlbad wird nun entfernt und nach · 1 Stunde weiterfgerührt, wobei die Temperatur bis gegen Zimmertemperatur stieg. Das nicht verbrauchte Lithium wird abfiltriert und die Lösung auf Eis/Wasser gegossen und mit Aether extrahiert, der mit Kochsalzlösung gewaschen wird. Die vereinigten Extrakte werden mit Magnesiumsulfat getrocknet und am Rotationsverdampfer eingeengt. Man erhält so 20 g eines rötlichen Oels, das ohne weitere Reinigung weiter verarbeitet wird.

Das ölige Produkt wird in 143 ml Eisessig gelöst, 47 ml Wasser und 5 g p-Toluolsulfonsäure zugegeben und über Nacht gerührt. Zur Aufarbeitung wird wiederum auf Eiswasser gegossen, mit Aether extrahiert, mit Natriumcarbonatlösung und schliesslich mit Wasser neutral gewaschen. Nach dem Trocknen und Eindampfen erhält man 12 g eines rotbraunen Oels, das ohne weitere Reinigung weiter verarbeitet wird.

Das gesamte erhaltene Rohprodukt wird in 61 ml Aceton und 37 ml Dichlormethan gelöst und mit einem Ueberschuss an Jones-Reagenz oxydiert. Das Reaktionsgemisch wird auf Eiswasser gegossen und mit Aether extrahiert, der mit Wasser neutral gewaschen wird. Nach dem Trocknen mit Magnesiumsulfat und Einengen erhält man 10,9 g eines gelben Oeles, das an der 100-fachen Menge Kieselgel mit Toluol-Aethylacetat chromatographiert wird. Durch Kristallisation der entsprechenden Fraktionen aus Methylenchlorid-Aether erhält man reines 5-Oxo-1,5-seco-A-trinor-17α-pregnan-21,17-carbolacton. Schmelzpunkt 134-135°, $[\alpha]_D$ + 7° (Dioxan 0,2).

Das Ausgangsmaterial wird wie folgt hergestellt :

17β-t-Butoxy-1,5-seco-A-trinorandrost-9-en-5-on wird durch reduktive Methylierung in 17β-t-Butoxy-1,5-seco-A-trinorandrostan-5-on, Schmelzpunkt 128-129° (aus Aether/Hexan) übergeführt.

Der t-Butyläther wird mit Essigsäure/Perchlorsäure gespalten. Nachfolgende Verseifung mit Kaliumcarbonat in Methanol ergibt 17β-Hydroxy-1,5-seco-A-trinorandrostan-5-on, Schmelzpunkt 99-100° (aus Aether/Hexan).

Ketalisierung liefert 17β-Hydroxy-1,5-seco-A-trinor-androstan-5-on-äthylenacetal, Schmelzpunkt 168-169° (Aether/Hexan).

Nach der Oxydation mit Pyridiniumchlorchromat erhält man 1,5-Seco-A-trinorandrostan-5,17-dion-5-(äthylenacetal), Schmelzpunkt 140-141° (aus Methylenchlorid/Hexan).

Beispiel 42

In Analogie zu Beispiel 39 erhält man aus 5-Oxo-1,5-seco-A-trinor-17α-pregn-6-en-21,17-carbolacton das 17β-Hydroxy-17-(3-hydroxypropyl)-1,5-seco-A-trinorandrost-6-en-5-on. Schmelzpunkt 169-171° (Krist. aus Aceton) UV $\lambda = 231$ nm, $\varepsilon = 8\,170$, $[\alpha]_D^{25}$ − 90° (Dioxan 1,0 %)

Beispiel 43

2,5 g 5-Oxo-1,5-seco-A-trinor-17α-pregn-6-en-21,17-carbolacton und 14,77 g Triphenylmethylphosphoniumbromid werden in 50 ml Dimethylsulfoxid gelöst und bei Raumtemperatur unter Argon innert 10 Minuten mit einer Lösung von 4,64 g Kalium-tert-butoxylat in 50 ml Dimethylsulfoxid versetzt. Nach 15 Minuten wurde das Gemisch unter Kühlung mit 6N Salzsäure auf pH 2 bis 3 gebracht und 30 Minuten nachgerührt. Dann wurde auf Eis gegossen und mit Aether extrahiert, der mit Kochsalzlösung neutral gewaschen wurde. Nach dem Trocknen mit Magnesiumsulfat und Eindampfen erhielt man 11 g gelbes Oel, die an 500 g Kieselgel mit Toluol/Aethylacetat 19 : 1 chromatographiert werden. Man erhält so 650 mg 1,2-Seco-A-dinor-17α-pregna-2,6-dien-21,17-carbolacton, das aus Methanol kristallisiert. Schmelzpunkt 175-176°, UV $\lambda_{max}$ 232 nm, $\varepsilon = 22\,000$, $[\alpha]_D$ − 64° (Dioxan 0,2).

Beispiel 44

5-Oxo-1,5-seco-A-trinor-17α-pregn-9(11)-en-21,17-carbolacton, 150-151,5° (aus Methylenchlorid/Aether), $[\alpha]_D$ − 40° (Dioxan 0,5 %), erhält man aus 1,5-Seco-A-trinorandrost-9(11)-en-5,17-dion-cyclic-5-(äthylenacetal) analog Beispiel 41.

Das Ausgangsmaterial wurde wie folgt hergestellt :

17β-t-Butoxy-1,5-seco-A-trinorandrost-9-en-5-on wird in t-Butanol mit Kalium-t-butylat und Methyljodid methyliert. Dabei kann je nach den angewandten Bedingungen 17β-t-Butoxy-1,5-seco-A-trinorandrost-9(11)-en-5-on, Schmelzpunkt 118° (aus Methylenchlorid/Aether) oder 17β-t-Butoxy-6α-methyl-1,5-seco-A-trinorandrost-9(11)-en-5-on, Schmelzpunkt 147-148° (aus Methylenchlorid/Aether) als Hauptprodukt erhalten werden.

Spaltung des t-Butyläthers mit Essigsäure/Perchlorsäure und nachfolgende Verseifung mit Ka-

liumcarbonat in Methanol gibt 17β-Hydroxy-1,5-seco-A-trinorandrost-9(11)-en-5-on, Schmelzpunkt 119-120° (aus Aceton-Hexan).

Ketalisierung und nachfolgende Oxydation mit Pyridiniumchlorchromat ergibt, 1,5-Seco-A-trinorandrost-9(11)-en-5,17-dion-5-(äthylenacetal), Schmelzpunkt 171-173° (aus Methylenchlorid/Aceton).

Beispiel 45

In Analogie zu Beispiel 41 erhält man aus 6α-Methyl-1,5-seco-A-trinorandrostan-5,17-dion-5-(äthylenacetal) das 6α-Methyl-5-oxo-1,5-seco-A-trinor-17-α-pregnan-21,27-carbolacton, Schmelzpunkt 187-188° (aus Methylenchlorid/-Hexan), $[\alpha]_D$ + 3° (Dioxan 0,5).

Das Ausgangsmaterial wurde wie folgt hergestellt :

17β-t-Butoxy-1,5-seco-A-trinorandrostan-5-on wird mit Lithiumdiisopropylamid-Methyljodid methyliert. Man erhält 17β-t-Butoxy-6α-methyl-1,5-seco-A-trinorandrostan-5-on, Schmelzpunkt 140-141°.

Spaltung des t-Butyläthers mit Essigsäure/Perchlorsäure und nachfolgende Verseifung ergibt 17β-Hydroxy-6α-methyl-1,5-seco-A-trinor-androstan-5-on, Schmelzpunkt 124-125°, aus dem durch Ketalisierung 17β-Hydroxy-6α-methyl-1,5-seco-A-trinorandrostan-5-on-äthylenacetal, Schmelzpunkt 192-193° erhalten wird.

Durch Oxydation mit Jones-Reagenz erhält man schliesslich 6α-Methyl-1,5-seco-A-trinorandrostan-5,17-dion-5-(äthylenacetal).

Beispiel 46

An Analogie zu Beispiel 42 erhält man aus 6α-Methyl-1,5-seco-A-trinorandrost-9(11)-en-5,17-dion-5-(äthylenacetal) das 6α-Methyl-5-oxo-1,5-seco-A-trinorpregn-9(11)-en-21,17-carbolacton, Schmelzpunkt 173-175° (aus Methylenochlorid/Aether), $[\alpha]_D$ − 41° (Dioxan 0,5).

Das Ausgangsmaterial wird wie folgt hergestellt :

Aus 17β-t-Butoxy-6α-methyl-1,5-seco-A-trinorandrost 9(11)-en-5-on erhält man durch Spaltung des t-Butyläthers mit Essigsäure/Perchlorsäure und nachfolgende Verseifung mit methanolischer Kaliumhydroxydlösung das 17β-Hydroxy-6α-methyl-1,5-seco-A-trinorandrost-9(11)-en-5-on, Schmelzpunkt 90-92° daraus durch Ketalisierung das 17β-Hydroxy-6α-methyl-1,5-seco-A-trinorandrost-9(11)-en-5-on-äthylenacetal. Schmelzpunkt 180-182°.

Jones-Oxydation ergibt 6α-Methyl-1,5-seco-A-trinorandrost-9(11)-en-5,17-dion-5-(äthylenacetal), Schmelzpunkt 128-129°.

Beispiel 47

In Analogie zu Beispiel 1 erhält man aus 5-Oxo-1,5-seco-D-homo-A-trinor-17aξ-pregnan-21,17a-carbolacton das 5-Oxo-1,5-seco-D-homo-A-trinor-17aξ-pregn-6-en-17a-carbolacton (Isomer A). Schmelzpunkt 227-228° (aus Aceton/Hexan). UV λ = 229 nm, ε = 9 300. $[\alpha]_D$ − 113° (Dioxan 0,2 %) sowie als Nebenprodukt das 6-Brom-6-oxo-1,5-seco-D-homo-A-trinor-17aξ-pregn-6-en-21,17a-carbolacton (Isomer A).

Aus dem entsprechenden Isomer B erhält man das 5-Oxo-1,5-seco-D-homo-A-trinor-17aξ-pregn-6-en-21,17a-carbolacton, (Isomer B), Schmelzpunkt 167-168° (aus Aceton/Hexan), UV λ = 229 nm, ε = 9 900, $[\alpha]_D$ − 92° (Dioxan 0,2) sowie als Nebenprodukt das 6-Brom-5-oxo-1,5-seco-D-homo-A-trinor-17aξ-pregn-6-en-21,17a-carbolacton (Isomer B), Schmelzpunkt 190-191° (aus Aceton/Hexan), UV λ = 254 nm, ε = 7 800, $[\alpha]_D$ − 10° (Dioxan 0,2).

Das Ausgangsmaterial 5-Oxo-1,5-seco-D-homo-A-trinor-17aξ-pregnan-21,17a-carbolacton wird wie folgt hergestellt :

1,5-Seco-A-trinorandrostan-5,17-dion-5-(äthylenacetal) wird mit Trimethylsulfoniummethoxysulfat und Kalium-t-butylat in Dimethylformamid zum Spiro [oxiran-2,17′ (beta 1)-[1,5] seco-A-trinorandrostan]-5′-on-äthylenacetal, Schmelzpunkt 131-134° (Methanol) umgesetzt.

Umsetzung mit Ammoniak gibt das 17-(Aminomethyl)-5,5-(äthylendioxy)-1,5-seco-A-trinorandrostan-17β-ol, Schmelzpunkt 193-196° (Rohprodukt), aus dem durch Tiffencan-Demjanow Ringerweiterung das 1,5-Seco-A-trinor-D-homoandrostan-5,17a-dion-5-(äthylenacetal), Schmelzpunkt 137-138° (Aceton/Hexan) erhalten wird.

Daraus erhält man durch Lithium-Grignard mit 3-Brom-propionaldehyd-dimethylacetal, Spaltung der beiden Acetale mit wässriger Essigsäure und Jones-Oxydation die beiden diastereomeren Lactone 5-Oxo-1,5-seco-D-homo-A-trinor-17aξ-pregnan-21,17a-carbolacton.

Isomer A, Schmelzpunkt 200,5-201° (aus Aceton/Hexan), $[\alpha]_D$ + 28° (Dioxan 0,5 %).

Isomer B, Schmelzpunkt 222-223° (aus Aceton/Hexan), $[\alpha]_D$ + 24° (Dioxan 0,5 %).

Beispiel 48

5 g 5β-Hydroxy-6,6-dimethyl-1,5-seco-A-trinorandrost-9(11)-en-17-on werden in 100 ml Tetrahydrofuran gelöst and unter Argon werden 1,25 g entfettete Lithiumdraht-Stücke zugegeben. Dann werden bei

ca. – 25° innert 2 Stunden 21,5 g 3-Brompropionaldehyd-dimethylacetal zugetropft. Nach beendeter Zugabe wird noch eine weitere Stunde bei dieser Temperatur gerührt, worauf man das Kühlbad entfernt und noch eine Stunde nachrührt, wobei die Temperatur bis gegen 20° ansteigt. Uebliche Aufarbeitung liefert 8,4 g Oel, das direkt weiter verabeitet wird.

Das gesamte so erhaltene Rohprodukt (8,4 g) wird in 30 ml Eisessig gelöst und 12,7 ml Wasser zugegeben. Nach Stehenlassen über Nacht wird aufgearbeitet. Man erhält 6,9 g Rohprodukt.

Das gesamte erhaltene Rohprodukt wird in 40 ml Aceton gelöst und bei Zimmertemperatur mit 10 ml Jones-Reagenz oxydiert. Das Rohprodukt (7,1 g) wird an 700 g $SiO_2$ mit Toluol-Aethylacetat chromatographiert. Die entsprechenden Fraktionen werden zusammengenommen und aus Aceton-Hexan kristallisiert. Man erhält so reines 6,6-Dimethyl-5-oxo-1,5-seco-A-trinor-17α-pregnen-9(11)-en-21,17-carbolacton von Schmelzpunkt 139-141°, $[\alpha]_D$ – 109° (Dioxan 0,15).

Das Ausgangsmaterial wird wie folgt hergestellt :

17β-t-Butoxy-6α-methyl-1,5-seco-A-trinorandrost-9(11)-en-5-on wird mit Lithiumdiisopropylamid/Methyljodid zum 17β-t-Butoxy-6,6-dimethyl-1,5-seco-A-trinorandrost-9(11)-en-5-on, Schmelzpunkt 93,5-95,5° methyliert.

Lithiumaluminiumhydrid-Reduktion gibt 17β-t-Butoxy-6,6-dimethyl-1,5-seco-A-trinorandrost-9(11)-en-5β-ol, Schmelzpunkt 163-164°.

Acetylierung mit Pyridin/Acetanhydrid bei erhöhter Temperatur liefert 17β-t-Butoxy-6,6-dimethyl-1,5-seco-A-trinorandrost-9(11)-en-5β-ol-acetat, Schmelzpunkt 129-130°.

Spaltung des t-Butyläthers mit Essigsäure/Perchlorsäure und nachfolgende Verseifung mit Kaliumcarbonat in Methanol ergibt 5β-Acetoxy-6,6-dimethyl-1,5-seco-A-trinorandrost-9(11)-en-17β-ol. Schmelzpunkt 134-135°.

Jones-Oxydation gibt das 17-Keton 5β-Acetoxy-6,6-dimethyl-1,5-seco-A-trinorandrost-9(11)-en-17-on, Schmelzpunkt 135,5-136° der noch durch Kochen in methanolischer Kalilauge verseift wird zum 5β-Hydroxy-6,6-dimethyl-1,5-seco-A-trinorandrost-9(11)-en-17-on. Schmelzpunkt 175-175,5°.

## Beispiel 49

In Analogie zu Beispiel 36 erhält man aus 6,6-Dimethyl-5-oxo-1,5-seco-A-trinor-17α-pregn-9(11)-en-21,17-carbolacton das Kalium 17-hydroxy-6,6-dimethyl-5-oxo-1,5-seco-A-trinor-17α-pregna-9(11)-en-21-carboxylat. Schmelzpunkt 261-263°, $[\alpha]_D$ – 67° (EtOH, c = 0,5 %).

## Beispiel 50

In Analogie zu Beispiel 48 wird aus 5β-Hydroxy-6,6-dimethyl-1,5-seco-A-trinorandrostan-17-on das 6,6-Dimethyl-5-oxo-1,5-seco-A-trinor-17α-pregnan-21,17-carbolacton erhalten. Schemelzpunkt 126-129° (aus Aceton/Hexan), $[\alpha]_D$ – 58° (Dioxan c = 0,2).

Das Ausgangsmaterial wird wie folgt hergestellt :

17β-t-Butoxy-6α-methyl-1,5-seco-A-trinorandrostan-5-on mit Lithiumdiisopropylamid/Methyljodid zum 17β-t-butoxy-6,6-dimethyl-1,5-seco-A-trinor-androstan-5-on, Schmelzpunkt 123-124° methyliert.

Reduktion des 5-Ketons mit Natriumborhydrid und nachfolgende Acetylierung liefert 5β-Acetoxy-17β-t-Butoxy-6,6-dimethyl-1,5-seco-A-trinorandrostan, Schmelzpunkt 117-118°.

Spaltung des t-Butyläthers und nachfolgende Behandlung mit Kaliumcarbonat in Methanol führt zum 5β-Acetoxy-6,6-dimethyl-1,5-seco-A-trinorandrostan-17β-ol, Schmelzpunkt 149-151°.

Jones-Oxydation ergibt 5β-Acetoxy-6,6-dimethyl-1,5-seco-A-trinorandrostan-17-on, Schmelzpunkt 132-133° aus dem durch Verseifung das 5β-Hydroxy-6,6-dimethyl-1,5-seco-A-trinorandrostan-17-on, Schmelzpunkt 183-184° erhalten wird.

## Beispiel 51

In Analogie zu Beispiel 48 erhält man aus 6,6-dimethyl-5-oxo-1,5-seco-A-trinor-17α-pregnan-21,17-carbolacton das Kalium 17-hydroxy-6,6-dimethyl-5-oxo-1,5-seco-A-trinor-17α-pregnan-21-carboxylat, Schmelzpunkt 274-284° (Isopropanol), $[\alpha]_D$ – 38° (Methanol 0,5 %).

## Beispiel A

Eine Tablette zur oralen Verabreichung kann die folgende Zusammensetzung aufweisen :

| | |
|---|---|
| Wirkstoff, z. B. 5-Oxo-A-tetranor-17α-pregna-9,11-dien-21,17-carbolacton | 25 mg |
| Maisstärke | 100 mg |
| Laktose | 50 mg |
| Polyvinylpyrrolidon | 15 mg |
| Magnesiumstearat | 2 mg |

Beispiel B

Eine Kapsel zur oralen Verabreichung kann die folgende Zusammensetzung aufweisen :

| | |
|---|---|
| Wirkstoff z. B. 5-Oxo-A-tetranor-17α-pregna-9,11-dien-21,17-carbolacton | 25 mg |
| Maisstärke | 125 mg |
| Laktose | 125 mg |

**Patentansprüche** (für die Vertragsstaaten : BE, CH, DE, FR, GB, IT, LI, LU, NL, SE)

1. deA-Steroide der Formel

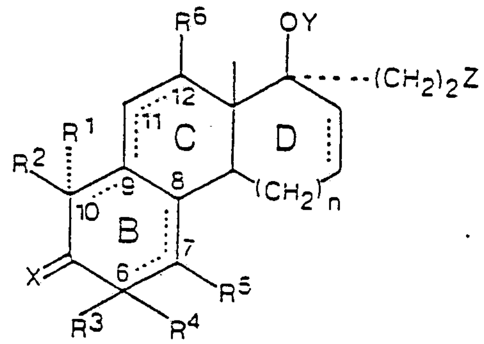

(I)

worin $R^1$ und $R^2$ Wasserstoff oder $C_{1-5}$-Alkyl ; $R^3$ Wasserstoff, Methyl oder Halogen ; $R^4$ Wasserstoff oder, falls $R^3$ Methyl ist, Wasserstoff oder Methyl ; $R^5$ Wasserstoff, Cyano, $C_{1-5}$-Alkoxy, oder einen Rest $SR^7$ ; oder $R^3$ und $R^5$ zusammen Methylen ; $R^6$ Wasserstoff oder $C_{1-7}$-Alkanoylthio ; $R^7$ $C_{1-7}$-Alkanoyl, $C_{1-8}$-Alkyl, Benzyl oder Phenäthyl ; X Oxo oder Methylen ; Y Wasserstoff ; Z Hydroxymethyl oder eine Gruppe —COA ; A Hydroxy ; oder Y und A zusammen eine O-C-Bindung bedeuten ; n 0 oder 1 ist und die punktierten ringständigen Bindungen fakultativ sind ; wobei jeder Ring höchstens eine Doppelbindung enthalten soll ; der Ring B 9(10)-ungesättigt ist, wenn der Ring C 11(12)-ungesättigt ist ; mindestens ein Rest $R^5$ oder $R^6$ Wasserstoff ist ; $R^5$ Wasserstoff ist, wenn $R^4$ Methyl ist ; $R^5$ Wasserstoff ist oder $R^3$ und $R^5$ zusammen Methylen sind, und $R^6$ Wasserstoff ist, wenn Z Carboxy ist ; $R^3$ Wasserstoff, Methyl oder Halogen ist und $R^4$ abwesend ist, wenn eine 6(7)- oder 7(8)-Doppelbindung vorliegt ; die 6(7)- und 11(12)-Bindung gesättigt ist, wenn $R^6$ $C_{1-7}$-Alkanoylthio ist ; und $R^1$ abwesend und $R^2$ $C_{1-5}$-Alkyl ist, wenn eine 9(10)-Doppelbindung vorliegt, die Ringe C und D gesättigt sind, n = 0 ist und Z eine Gruppe —COA ist, oder Salze von Verbindungen der Formel I, in denen Z eine Carboxylgruppe ist, zur Verwendung als Pharmazeutika.

2. deA-Steroide gemäss Anspruch 1 der Formel

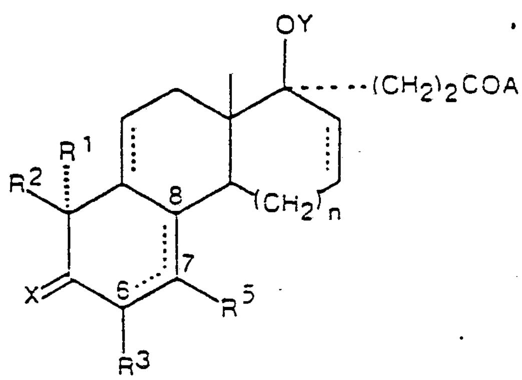

(I-1)

worin $R^1$, $R^2$, $R^3$, $R^5$, A, Y, n und die punktierten Bindungen die in Anspruch 1 angegebene Bedeutung haben, zur Verwendung als Pharmazeutika.

3. deA-Steroide der Formel I oder I-1 gemäss Anspruch 1 oder 2, in denen mindestens ein Rest $R^1$ oder $R^2$ $C_1$-$_5$Alkyl ist, zur Verwendung als Pharmazeutika.

4. deA-Steroide der Formel I oder I-1 gemäss Anspruch 1 oder 2, in denen die Ringe B und C ungesättigte sind, zur Verwendung als Pharmazeutika.

5. deA-Steroide der Formel I gemäss Anspruch 1, in denen Z Hydroxymethyl ist, zur Verwendung als Pharmazeutika.

6. 5-Oxo-A-tetranor-17α-pregna-9,11-dien-21,17-carbonlacton, zur Verwendung als Pharmazeutikum.

7. DeA-Steroide der Formel

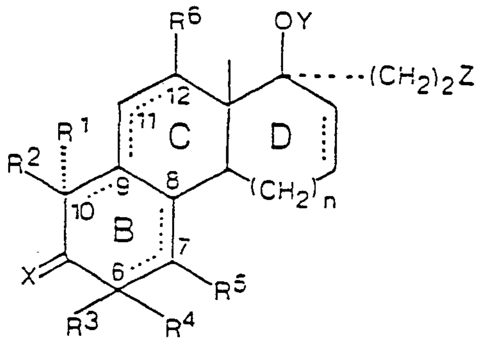

(I)

worin $R^1$ und $R^2$ Wasserstoff oder $C_{1-5}$-Alkyl ; $R^3$ Wasserstoff, Methyl oder Halogen ; $R^4$ Wasserstoff oder, falls $R^3$ Methyl ist, Wasserstoff oder Methyl ; $R^5$ Wasserstoff oder Methyl ; $R^5$ Wasserstoff, Cyano, $C_{1-5}$-Alkoxy, oder einen Rest $SR^7$ ; oder $R^3$ und $R^5$ zusammen Methylen ; $R^6$ Wasserstoff oder $C_{1-7}$-Alkanoylthio ; $R^7$ $C_{1-7}$-Alkanoyl, $C_{1-8}$-Alkyl, Benzyl oder Phenäthyl ; X Oxo oder Methylen ; Y Wasserstoff ; Z Hydroxymethyl oder eine Gruppe —COA ; A Hydroxy ; oder Y und A zusammen eine O-C-Bindung bedeuten ; n 0 oder 1 ist und die punktierten ringständigen Bindungen fakultativ sind ; wobei jeder Ring höchstens eine Doppelbindung enthalten soll ; der Ring B 9(10)-ungesättig ist, wenn der Ring C 11(12)-ungesättigt ist ; mindestens ein Rest $R^5$ oder $R^6$ Wasserstoff ist ; $R^5$ Wasserstoff ist, wenn $R^4$ Methyl ist ; $R^5$ Wasserstoff ist oder $R^3$ und $R^5$ zusammen Methylen sind, und $R^6$ Wasserstoff ist, wenn Z Carboxy ist ; $R^3$ Wasserstoff, Methyl oder Halogen ist und $R^4$ abwesend ist, wenn eine 6(7)- oder 7(8)-Doppelbindung vorliegt ; die 6(7)- und 11(12)-Bindung gesättigt ist, wenn $R^6$ thio ist ; und die Ringe B und C ungesättigt sind, und Salze von Verbindungen der Formel I, in denen Z eine Carboxylgruppe ist.

8. DeA-Steroide gemäss Anspruch 8 der Formel

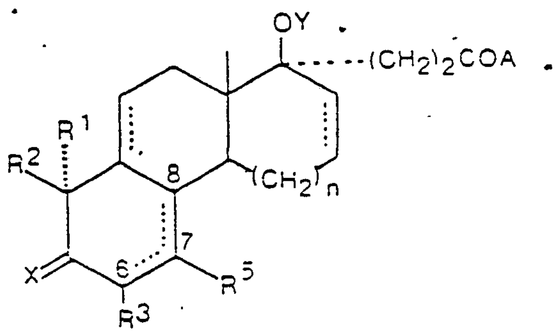

(IA-1)

worin $R^1$, $R^2$, $R^3$, $R^5$, A, X, A, Y, n und die punktierten Bindungen die in Anspruch 8 angegebene Bedeutung haben.

9. DeA-Steroide gemäss Anspruch 8 oder 9, in denen mindestens ein Rest $R^1$ oder $R^2$ $C_{1-5}$-Alkyl ist.

10. DeA-Steroide gemäss einem der Ansprüche 8, 9 oder 10, in denen Z Hydroxymethyl ist.

11. 5-Oxo-A-tetranor-17α-pregna-9,11-dien-21,17-carbolacton

12. Verfahren zur Herstellung der in Anspruch 7 definierten deA-Steroide, dadurch gekennzeichnet, dass man

a) in einer Verbindung der Formel

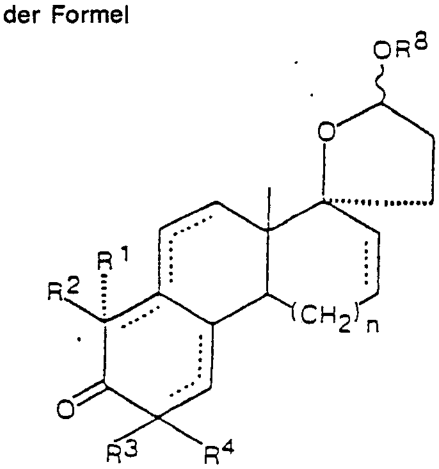

(II)

die Gruppe $OR^8$ zur Oxogruppe oxydiert ; oder

b) eine Verbindung der Formel

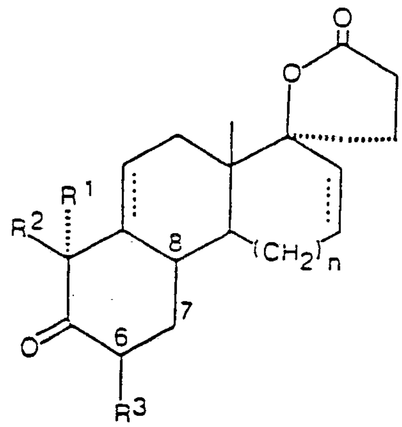

(Ia)

oder die entsprechende Säure oder ein Salz davon in 6(7)- oder 7(8)-Stellung dehydriert ; oder

c) eine Verbindung der Formel.

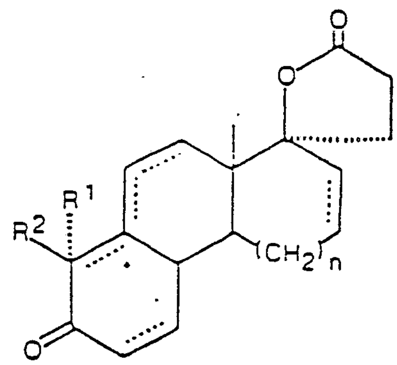

(Ib)

oder die entsprechende Säure oder ein Salze davon halogeniert ; oder

d) eine Verbindung der Formel

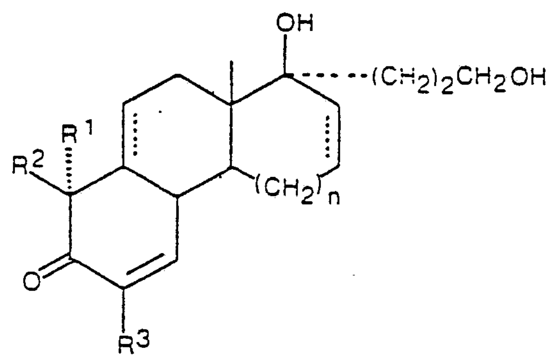

(Id)

mit einer Verbindung $R^7SH$, einem $C_{1-5}$-Alkanol, oder Cyanwasserstoff umsetzt ; oder

e) eine Verbindung der Formel

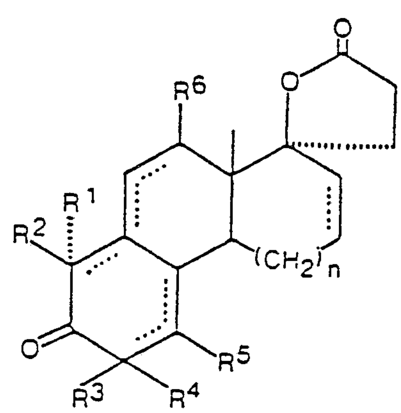

(Ih)

die entsprechende Säure oder ein Salz davon mit einer. Methylen-phosphoniumverbindung nach Wittig umsetzt ; oder

f) in einer Verbindung der Formel

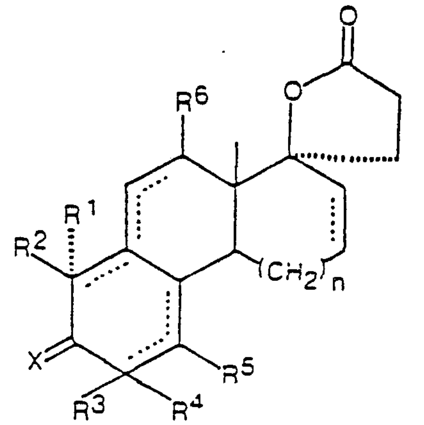

(Ii)

den Lactonring öffent, oder

g) in einer Verbindung der Formel I, in der Z die Gruppe COA darstellt, diese Gruppe zur Hydroxylmethylgruppe reduziert ; wobei in den obigen Formeln $R^1$, $R^2$, $R^3$, $R^4$, X, n und die punktierten ringständigen Bindungen die oben angegebene Bedeutung haben und $R^8$ Wasserstoff oder $C_{1-8}$ Alkyl darstellt.

**Patentansprüche** (für den Vertragsstaat AT)

1. Verfahren zur Herstellung von deA-Steroiden der Formel

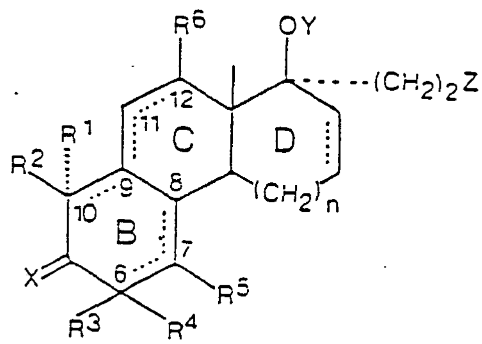

(IA)

worin $R^1$ und $R^2$ Wasserstoff oder $C_{1-5}$-Alkyl ; $R^3$ Wasserstof, Methyl oder Halogen ; $R^4$ Wasserstoff oder, falls $R^3$ Methyl ist, Wasserstoff oder Methyl ; $R^5$ Wasserstoff, Cyano, $C_{1-5}$-Alkoxy, oder einen Rest $SR^7$ ; oder $R^3$ und $R^5$ zusammen Methylen ; $R^6$ Wasserstoff oder Acylthio ; $R^7$ $C_{1-7}$-Alkanoyl, $C_{1-8}$-Alkyl, Benzyl oder Phenäthyl ; X Oxo oder Methylen ; Y Wasserstoff ; Z Hydroxymethyl oder eine Gruppe —COA ; A Hydroxy ; oder Y und A zusammen eine O-C-Bindung bedeuten ; 0 oder 1 ist und die punktierten ringständigen Bindungen fakultativ sind ; wobei jeder Ring höchstens eine Doppelbindung enthalten soll ; der Ring B 9(10)-ungesättigt ist, wenn der Ring C 11(12)-ungesättigt ist ; mindestens ein Rest $R^5$ oder $R^6$ Wasserstoff ist ; $R^5$ Wasserstoff ist, wenn $R^4$ Methyl ist ; $R^5$ Wasserstoff ist oder $R^3$ und $R^5$ zusammen Methylen sind, und $R^6$ Wasserstoff ist, wenn Z Carboxy ist ; $R^3$ Wasserstoff, Methyl oder Halogen ist und $R^4$ abwesend ist, wenn eine 6(7)- oder 7(8)-Doppelbindung vorliegt ; die 6(7)- und 11(12)-Bindung gesättigt ist, wenn $R^6$ $C_{1-7}$-Alkanoylthio ist ; und die Ringe B und C ungesättigt sind, dadurch gekennzeichnet, dass man

a) in einer Verbindung der Formel

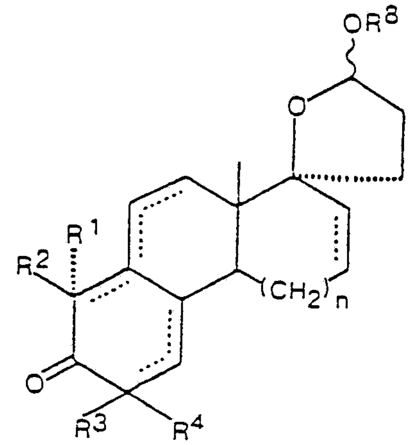

(II)

die Gruppe $OR^8$ zur Oxogruppe oxydiert ; oder

b) eine Verbindung der Formel

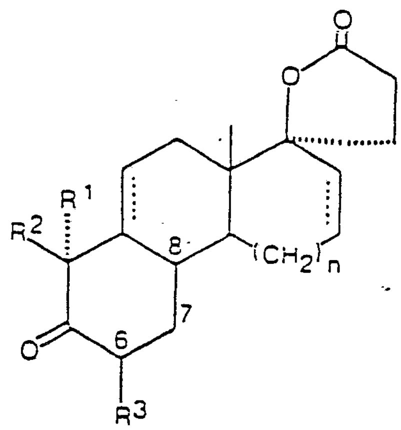

(Ia)

oder die entsprechende Säure oder ein Salz davon in 6(7)- oder 7(8)-Stellung dehydriert ; oder

c) eine Verbindung der Formel

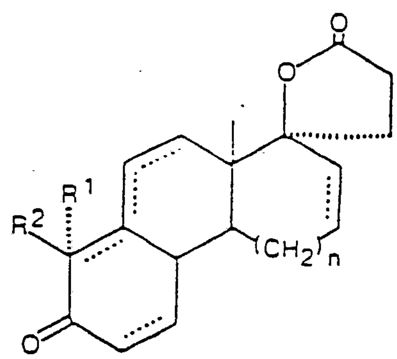

(Ib)

oder die entsprechende Säure oder ein Salz davon halogeniert ; oder

d) eine Verbindung der Formel

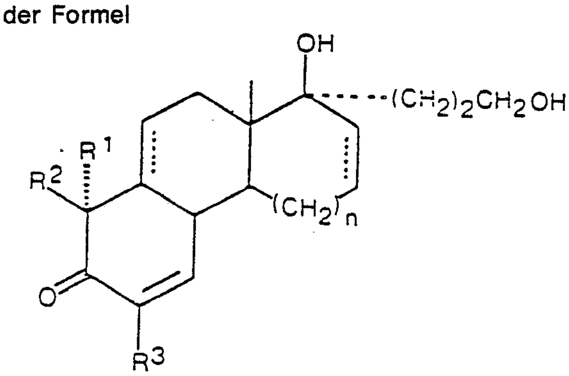

(Id)

mit einer Verbindung $R^7SH$, einem $C_{1\text{-}5}$-Alkanol, oder Cyanwasserstoff umsetzt ; oder

e) eine Verbindung der Formel

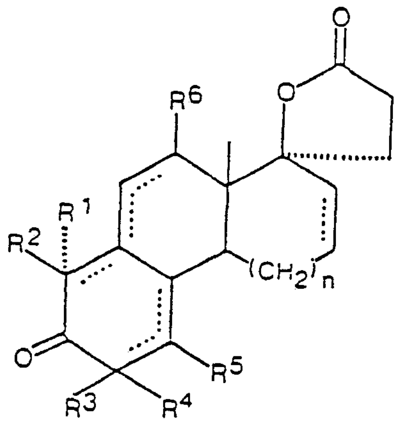

(Ih)

die entsprechende Säure oder ein Salz davon mit einer Methylen-phosphoniumverbindung nach Wittig umsetzt ; oder

f) in einer Verbindung der Formel

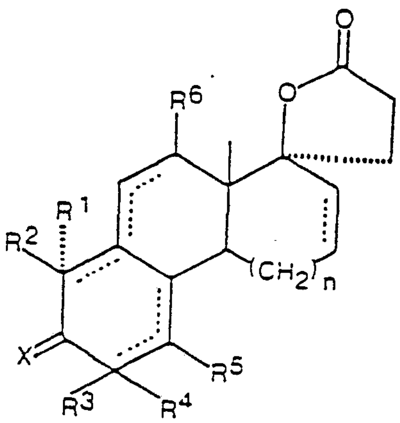

(Ii)

den Lactonring öffnet ; oder

g) in einer Verbindung der Formel I, in der Z die Gruppe COA darstellt, diese Gruppe zur Hydroxylmethylgruppe reduziert ; wobei in den obigen Formeln $R^1$, $R^2$, $R^3$, $R^4$, X, n und die punktierten ringständigen Bindungen die oben angegebene Bedeutung haben und $R^8$ Wasserstoff oder Alkyl darstellt.

2. Verfahren nach Anspruch 1, dadurch gekennzeichnet, dass man deA-Steroide der Formel

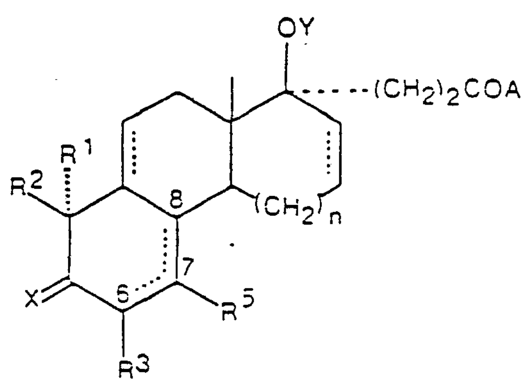

(IA-1)

worin $R^1$, $R^2$, $R^3$, $R^5$, A, Y, n und die punktierten Bindungen die in Anspruch 1 angegebene Bedeutung haben, herstellt.

3. Verfahren nach den Ansprüchen 1 oder 2, dadurch gekennzeichnet, dass man deA-Steroide der Formel I A oder I A-1 herstellt, in denen mindestens in Rest $R^1$ oder $R^2$ $C_{1\text{-}5}$-Alkyl ist.

4. Verfahren nach den Ansprüchen 1, 2 oder 3, dadurch gekennzeichnet, dass man deA-Steroide der Formeln I A oder IA-1 herstellt, in denen die Ringe B und C ungesättigt sind.

5. Verfahren nach einem der Ansprüche 1, 3 oder 4, dadurch gekennzeichnet, dass man deA-Steroide der Formel 1 A herstellt, in denen Z Hydroxymethyl ist.

6. Verfahren nach Anspruch 1, dadurch gekennzeichnet, dass man 5-Oxo-A-tetranor-17α-pregna-9,11-dien-21,17-carbolacton herstellt.

**Claims** (for the Contracting States : BE, CH, DE, FR, GB, IT, LI, LU, NL, SE)

1. DeA-steroids of the formula

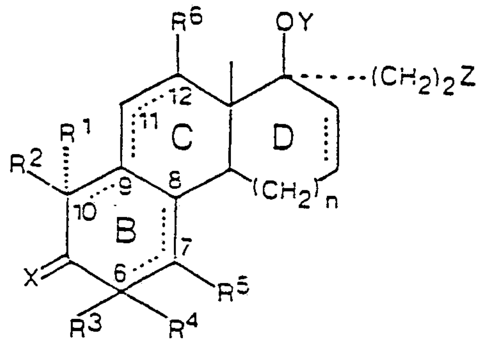

(I)

wherein $R^1$ and $R^2$ signify hydrogen or $C_{1-5}$-alkyl ; $R^3$ signifies hydrogen, methyl or halogen ; $R^4$ signifies hydrogen or, where $R^3$ is methyl, $R^4$ signifies hydrogen or methyl ; $R^5$ signifies hydrogen, cyano, $C_{1-5}$-alkoxy or a residue $SR^7$ ; or $R^3$ and $R^5$ together signify methylene ; $R^6$ signifies hydrogen $C_{1-7}$-alkanoylthio ; $R^7$ signifies $C_{1-7}$-alkanoyl, $C_{1-8}$-alkyl, benzyl or phenethyl ; X signifies oxo or methylene ; Y signifies hydrogen ; Z signifies hydroxymethyl or a group —COA ; A signifies hydroxy ; or Y and A together signify a O-C bond ; n is 0 or 1 and the dotted bonds in the ring are optional ; whereby each ring contains at most one double bond ; the ring B is 9(10)-unsaturated when the ring C is 11(12)-unsaturated ; at least one residue $R^5$ or $R^6$ is hydrogen ; $R^5$ is hydrogen when $R^4$ is methyl ; $R^5$ is hydrogen or $R^3$ and $R^5$ together are methylene and $R^6$ is hydrogen when Z is carboxy ; $R^3$ is hydrogen, methyl or halogen and $R^4$ is absent when a 6(7)- or 7(8)-double bond is present ; the 6(7)- and 11(12)-bond is saturated when $R^6$ is $C_{1-7}$-alkanoylthio ; and $R^1$ is absent and $R^2$ is $C_{1-5}$-alkyl when a 9(10)-double bond is present, the rings C and D are saturated, n is 0 and Z is a group —COA, or salts of compounds of formula I in which Z is a carboxyl group, for use as pharmaceuticals.

2. DeA-steroids in accordance with claim 1 of the formula

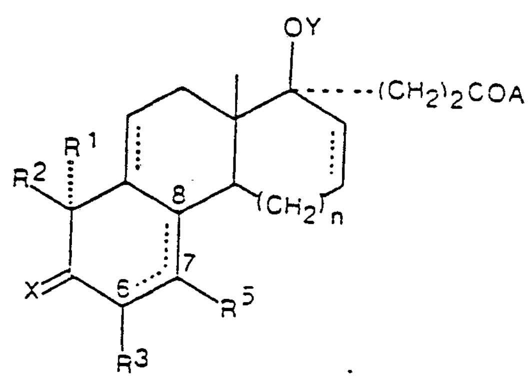

, (I-1)

wherein $R^1$, $R^2$, $R^3$, $R^5$, A, Y, n and the dotted bonds have the significance given in claim 1, for use as pharmaceuticals.

3. DeA-steroids of formula I or I-1 in accordance with claim 1 or 2, in which at least one residue $R^1$ or $R^2$ is $C_{1-5}$-alkyl, for use as pharmaceuticals.

4. DeA-steroids of formula I or I-1 in accordance with claim 1 or 2, in which the rings B and C are unsaturated, for use as pharmaceuticals.

5. DeA-steroids of formula I in accordance with claim 1, in which Z is hydroxymethyl, for use as pharmaceuticals.

6. 5-Oxo-A-tetranor-17α-pregna-9,11-diene-21,17-carbolactone, for use as a pharmaceutical.

7. DeA-steroids of the formula

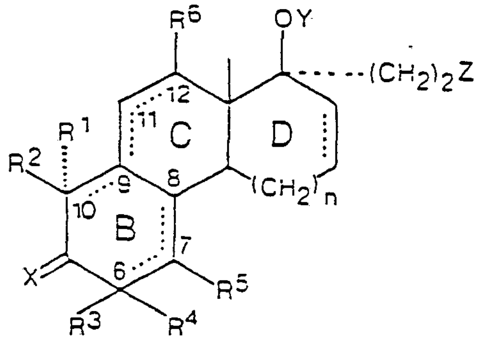

(I)

wherein $R^1$ and $R^2$ signify hydrogen or $C_{1-5}$-alkyl ; $R^3$ signifies hydrogen, methyl or halogen ; $R^4$ signifies hydrogen or, where $R^3$ is methyl, $R^4$ signifies hydrogen or methyl ; $R^5$ signifies hydrogen, cyano, $C_{1-5}$-alkoxy or a residue $SR^7$ ; or $R^3$ and $R^5$ together signify methylene ; $R^6$ signifies hydrogen or $C_{1-7}$-alkanoylthio ; $R^7$ signifies $C_{1-7}$-alkanoyl, $C_{1-6}$-alkyl, benzyl or phenethyl ; X signifies oxo or methylene ; Y signifies hydrogen ; Z signifies hydroxymethyl or a group —COA ; A signifies hydroxy ; or Y and A together signify a O-C bond ; n is 0 or 1 and the dotted bonds in the ring are optional ; whereby each ring contains at most one double bond ; the ring B is 9(10)-unsaturated when the ring C is 11(12)-unsaturated ; at least one residue $R^5$ or $R^6$ is hydrogen ; $R^5$ is hydrogen when $R^4$ is methyl ; $R^5$ is hydrogen or $R^3$ and $R^5$ together are methylene and $R^6$ is hydrogen when Z is carboxy ; $R^3$ is hydrogen, methyl or halogen and $R^4$ is absent when a 6(7)- or 7(8)-double bond is present ; the 6(7)- and 11(12)-bond is saturated when $R^6$ is $C_{1-7}$-alkanoylthio ; and the rings B and C are unsaturated, and salts of compounds of formula I in which Z is a carboxyl group.

8. DeA-steroids in accordance with claim 8 of the formula

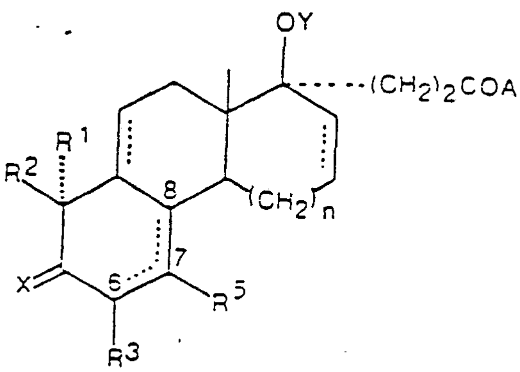

(IA-1)

wherein $R^1$, $R^2$, $R^3$, $R^5$, A, X, A, Y, n and the dotted bonds have the significance given in claim 8.

9. DeA-steroids in accordance with claim 8 or 9, in which at least one residue $R^1$ or $R^2$ is $C_{1-5}$-alkyl.

10. DeA-steroids in accordance with any one of claims 8, 9 or 10, in which Z is hydroxymethyl.

11. 5-Oxo-A-tetranor-17α-pregna-9,11-diene-21,17-carbolactone.

12. A process for the manufacture of the deA-steroids defined in claim 7, characterized by

a) oxidizing the group $OR^8$ in a compound of the formula

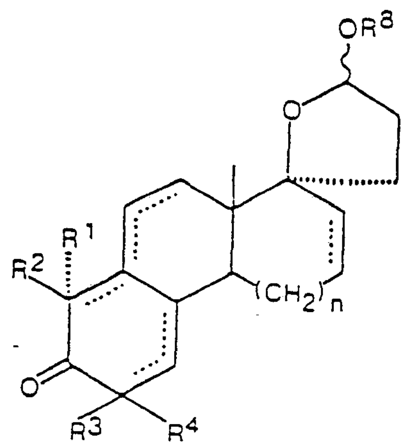

(II)

to the oxo group ; or

b) dehydrogenating a compound of the formula

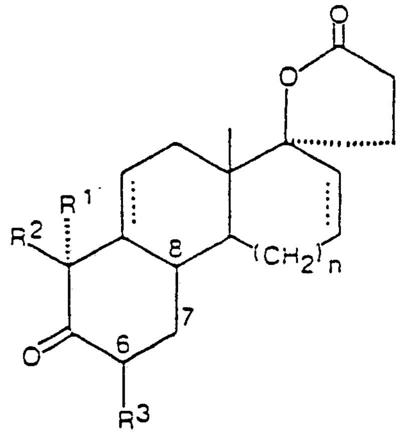

(Ia)

or the corresponding acid or a salt thereof in the 6(7)- or 7(8)-position ; or

c) halogenating a compound of the formula

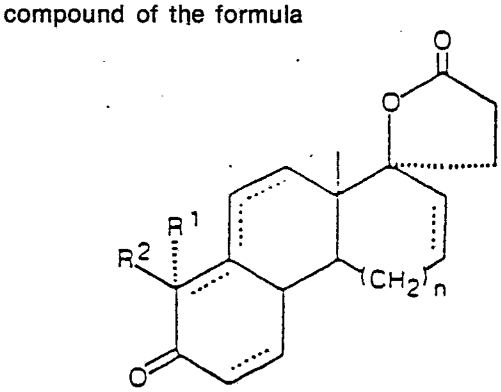

(Ib)

or the corresponding acid or a salt thereof ; or

d) reacting a compound of the formula

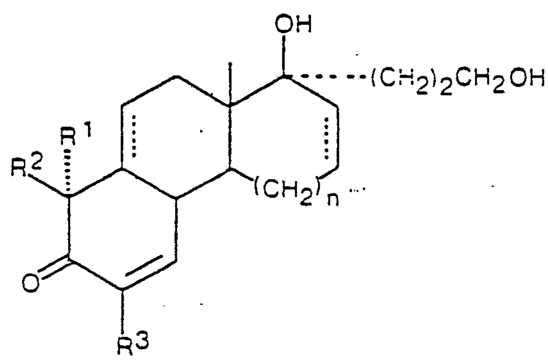

(Id)

with a compound $R^7SH$, a $C_{1-5}$-alkanol or hydrogen cyanide ; or

e) reacting a compound of the formula

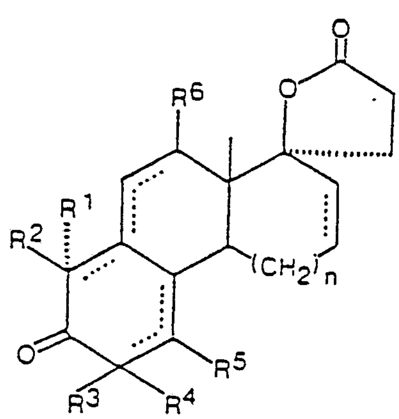

(Ih)

the corresponding acid or salt thereof with a methylenephosphonium compound according to Wittig ; or

f) opening the lactone ring in a compound of the formula

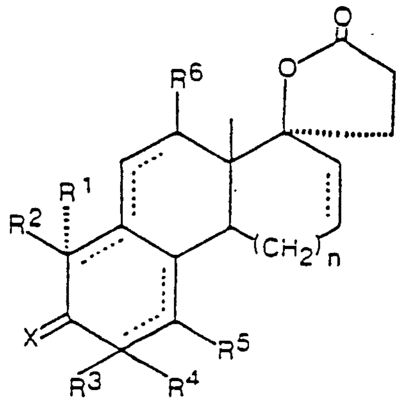

(Ii)

or

g) in a compound of formula I in which Z represents the group COA, reducing this group to the hydroxymethyl group ; whereby in the above formulae $R^1$, $R^2$, $R^3$, $R^4$, X, n and the dotted bonds in the ring have the significance given above and $R^8$ represents hydrogen or $C_{1-8}$-alkyl.

**Claims** (for the Contracting State AT)

1. A process for the manufacture of deA-steroids of the formula

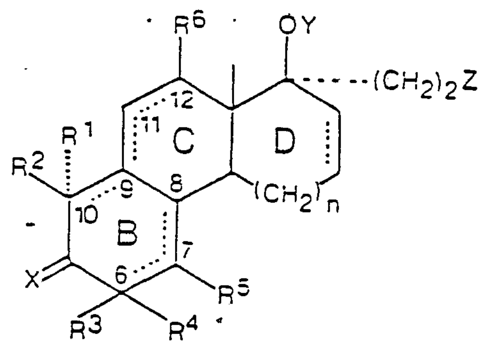

(IA)

wherein $R^1$ and $R^2$ signify hydrogen or $C_{1-5}$-alkyl ; $R^3$ signifies hydrogen, methyl or halogen ; $R^4$ signifies hydrogen or, where $R^3$ is methyl, $R^4$ signifies hydrogen or, where $R^3$ is methyl, $R^4$ signifies hydrogen or methyl ; $R^5$ signifies hydrogen, cyano, $C_{1-5}$-alkoxy or a residue $SR^7$ ; or $R^3$ and $R^5$ together signify methylene ; $R^6$ signifies hydrogen or $C_{1-7}$-alkanoylthio ; $R^7$ signifies $C_{1-7}$-alkanoyl, $C_{1-8}$-alkyl, benzyl or phenethyl ; X signifies oxo or methylene ; Y signifies hydrogen ; Z signifies hydroxymethyl or a group —COA ; A signifies hydroxy ; or Y and A together signify a O-C bond ; n is 0 or 1 and the dotted bonds in the ring are optional ; whereby each ring contains at most one double bond ; the ring B is 9(10)-unsaturated when the ring C is 11(12)-unsaturated ; at least one residue $R^5$ or $R^6$ is hydrogen ; $R^5$ is hydrogen when $R^4$ is methyl ; $R^5$ is hydrogen or $R^3$ and $R^5$ together are methylene and $R^6$ is hydrogen when Z is carboxy ; $R^3$ is hydrogen, methyl or halogen and $R^4$ is absent when a 6(7)- or 7(8)-double bond is present ; the 6(7)- and 11(12)-bond is saturated when $R^6$ is $C_{1-7}$-alkanoylthio ; and the rings B and C are unsaturated, characterized by

a) oxidizing the group $OR^8$ in a compound of the formula

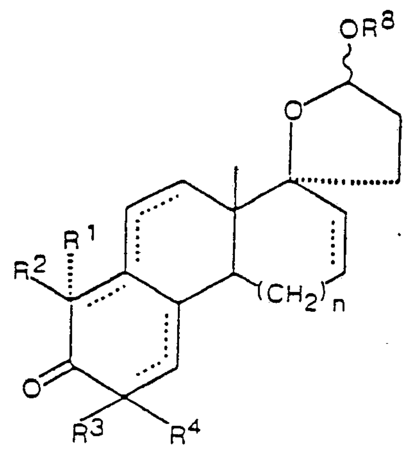

(II)

to the oxo group ; or

b) dehydrogenating a compound of the formula

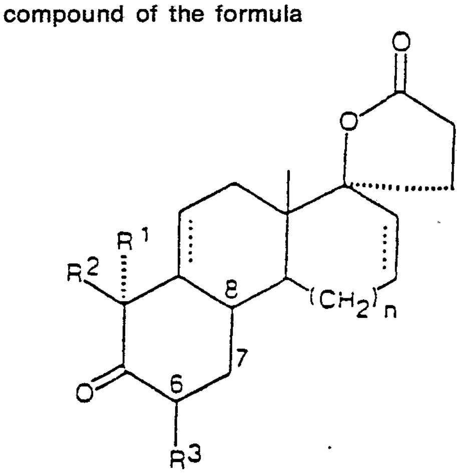

(Ia)

or the corresponding acid or a salt thereof in the 6(7)- or 7(8)-position ; or

c) halogenating a compound of the formula

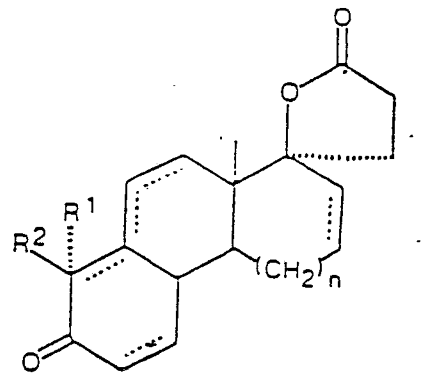

(Ib)

or the corresponding acid or a salt thereof ; or

d) reacting a compound of the formula

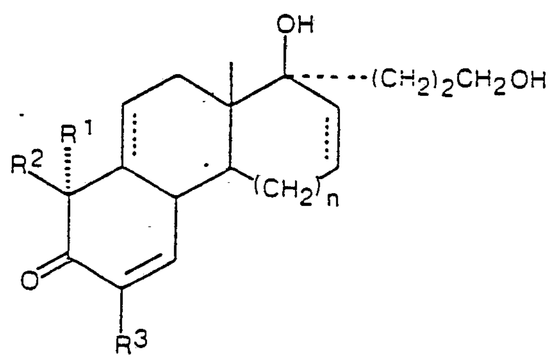

(Id)

with a compound $R^7SH$, a $C_{1-5}$-alkanol or hydrogen cyanide ; or

e) reacting a compound of the formula

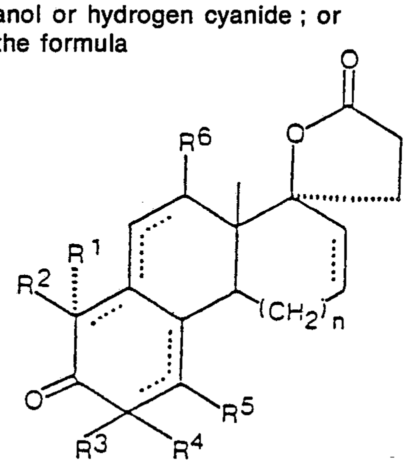

(Ih)

the corresponding acid or a salt thereof with a methylenephosphonium compound according to Wittig ; or

f) opening the lactone ring in a compound of the formula

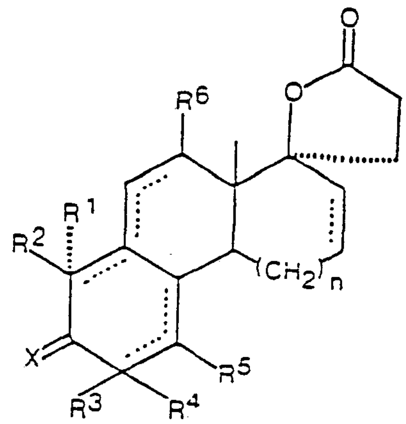

(Ii)

or

g) in a compound of formula I in which Z represents the group COA, reducing this group to the hydroxymethyl group ; whereby in the above formulae $R^1$, $R^2$, $R^3$, $R^4$, X, n and the dotted bonds in the ring have the significance given above and $R^8$ represents hydrogen or alkyl.

2. A process according to claim 1, characterized in that deA-Steroids of the formula

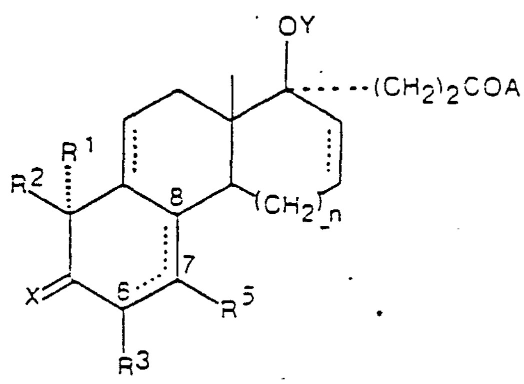

(IA-1)

wherein $R^1$, $R^2$, $R^3$, $R^5$, A, Y, n and the dotted bonds have the significance given in claim 1, are manufactured.

3. A process according to claims 1 or 2, characterized in that deA-steroids of formula IA or IA-1 in which at least one residue $R^1$ or $R^2$ is $C_{1\text{-}5}$-alkyl are manufactured.

4. A process according to claims 1, 2 or 3, characterized in that deA-steroids of formula IA or IA-1 in which the rings B and C are unsaturated are manufactured.

5. A process according to any one of claims 1, 3 or 4, characterized in that deA-steroids of formula IA in which Z is hydroxymethyl are manufactured.

6. A process according to claim 1, characterized in that 5-oxo-A-tetranor-17α-pregna-9,11-diene-21,17-carbolactone is manufactured.

**Revendications** (pour les Etats contractants : BE, CH, DE, FR, GB, IT, LI, LU, NL, SE)

1. Dés-A-stéroïdes de formule

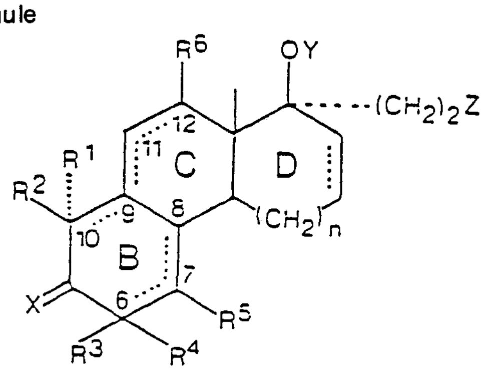

(I)

dans laquelle $R^1$ et $R^2$ représentent l'hydrogène ou un groupe alkyle en C1-C5 ; représente l'hydrogène, un groupe méthyle ou un halogène ; $R^4$ représente l'hydrogène, ou bien, lorsque $R^3$ représente un groupe méthyle, l'hydrogène ou un groupe méthyle ; $R^5$ représente l'hydrogène, un groupe cyano, alcoxy en C1-C5 ou un reste $SR^7$ ; ou bien $R^3$ et $R^5$ forment ensemble un groupe méthylène ; $R^6$ représente l'hydrogène ou un groupe alcanoylthio en C1-C7 ; $R^7$ représente un groupe alcanoyle en C1-C7, alkyle en C1-C8, benzyle ou phénéthyle ; X représente un groupe oxo ou méthylène ; Y représente l'hydrogène ; Z représente un groupe hydroxyméthyle ou un groupe —COA ; A représente un groupe hydroxy ; ou bien Y et A forment ensemble une liaison O-C ; n est égal à 0 ou 1 et, les liaisons cycliques figurant en pointillé sont facultatives ; chaque cycle contient au maximum une double liaison ; le cycle B est insaturé en 9 (10) lorsque le cycle C est insaturé en 11 (12) ; au moins un symbole $R^5$ ou $R^6$ représente l'hydrogène ; $R^5$ représente l'hydrogène lorsque $R^4$ représente un groupe méthyle ; $R^5$ représente l'hydrogène ou bien $R^3$ et $R^5$ forment ensemble le groupe méthylène et $R^6$ représente l'hydrogène lorsque Z représente un groupe carboxy ; $R^3$ représente l'hydrogène, un groupe méthyle ou un halogène et $R^4$ est absent lorsqu'il y a une double liaison 6 (7) ou 7 (8) ; la liaison 6 (7) et 11 (12) est saturée lorsque $R^6$ représente un groupe alcanoylthio en C1-C7 ; et $R^1$ est absent et $R^2$ représente un groupe alkyle en C1-C5 lorsqu'il y a une double liaison 9 (10), les cycles C et D sont saturés, n = 0 et Z représente un groupe —COA, ou les sels des composés de formule I dans lesquels Z représente un groupe carboxyle, pour l'utilisation en tant que produits pharmaceutiques.

2. Dés-A-stéroïdes selon la revendication 1, de formule

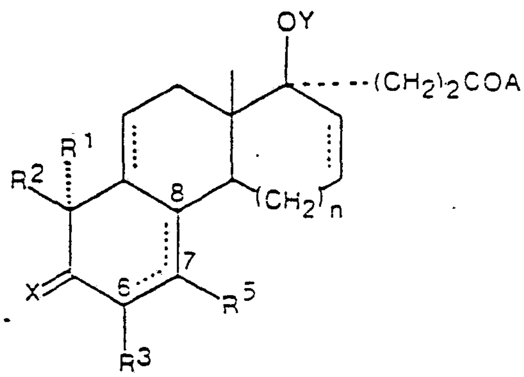

(I-1)

dans laquelle $R^1$, $R^2$, $R^3$, $R^5$, A, Y, n et les liaisons en pointillé ont les significations dans la revendication 1, pour l'utilisation en tant que produits pharmaceutiques.

3. Dés-A-stéroïdes de formule I ou I-1 selon la revendication 1 ou 2, dans lesquels l'un au moins des symboles $R^1$ ou $R^2$ représente un groupe alkyle en C1-C5, pour l'utilisation en tant que produits pharmaceutiques.

4. Dés-A-stéroïdes de formule I ou I-1 selon la revendication 1 ou 2 dans lesquels les cycles B et C sont insaturés, pour l'utilisation en tant que produits pharmaceutiques.

5. Dés-A-stéroïdes de formule I selon la revendication 1 dans lesquels Z représente un groupe hydroxyméthyle, pour l'utilisation en tant que produits pharmaceutiques.

6. La 5-oxo-A-tétranor-17alpha-prégna-9,11-diène-21,17-carboxylactone, pour l'utilisation en tant que produits pharmaceutiques.

7. Dés-A-stéroïdes de formule

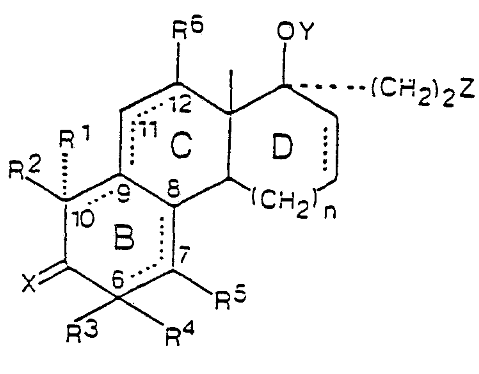

(I)

dans laquelle $R^1$ et $R^2$ représentent l'hydrogène ou un groupe alkyle en C1-C5 ; $R^3$ représente l'hydrogène, un groupe méthyle ou un halogène ; $R^4$ représente l'hydrogène ou bien, lorsque $R^3$ représente un groupe méthyle, l'hydrogène ou un groupe méthyle ; $R^5$ représente l'hydrogène, un groupe

cyano, alcoxy en C1-C5 ou un reste $SR^7$ ; ou bien $R^3$ et $R^5$ forment ensemble un groupe méthylène ; $R^6$ représente l'hydrogène ou un groupe alcanoylthio en C1-C7 ; $R^7$ représente un groupe alcanoyle en C1-C7, alkyle en C1-C8, benzyle ou phénéthyle ; X représente un groupe oxo ou méthylène ; Y représente l'hydrogène ; Z représente un groupe hydroxyméthyle ou un groupe —COA ; A représente un groupe hydroxy ; ou bien Y et A forment ensemble une liaison O—C ; n est égal à 0 ou 1 et les liaisons cycliques figurant en pointillé sont facultatives ; chaque cycle contient au maximum une double liaison ; le cycle B est insaturé en 9 (10) lorsque le cycle C est insaturé en 11 (12) ; au moins l'un des symboles $R^5$ et $R^6$ représente l'hydrogène ; $R^5$ représente l'hydrogène lorsque $R^4$ représente un groupe méthyle ; $R^5$ représente l'hydrogène, ou bien $R^3$ et $R^5$ forment ensemble un groupe méthylène, et $R^6$ représente l'hydrogène, lorsque Z représente un groupe carboxy ; $R^3$ représente l'hydrogène, un groupe méthyle ou un halogène et $R^4$ est absent lorsqu'il y a une double liaison 6 (7) ou 7 (8) ; la liaison 6 (7) et 11 (12) est saturée lorsque $R^6$ représente une fonction thio ; et les cycles B et C sont insaturés, et les sels des composés de formule I dans lesquels Z représente un groupe carboxyle.

8. Dés-A-stéroïdes selon la revendication 8, de formule

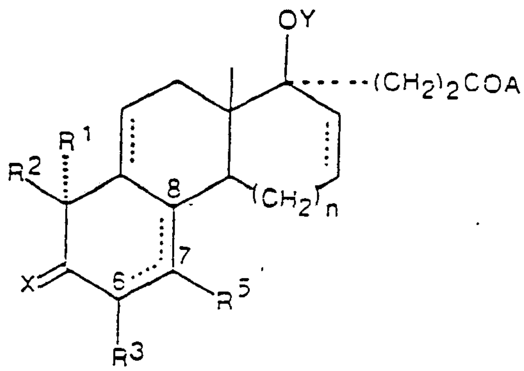

(IA-1)

dans laquelle $R^1$, $R^2$, $R^3$, $R^5$, A, X, A, Y, n et les liaisons en pointillé ont les significations indiquées dans la revendication 8.

9. Dés-A-stéroïdes selon la revendication 8 ou 9, dans lesquels l'un des symboles $R^1$ et $R^2$ représente un groupe alkyle en C1-C5.

10. Dés-A-stéroïdes selon l'une des revendications 8, 9 ou 10, dans lesquels Z représente un groupe hydroxyméthyle.

11. La 5-oxo-A-tétranor-17alpha-prégna-9,11-diène-21,17-carboxylactone.

12. Procédé de préparation des dés-A-stéroïdes définis dans la revendication 7, caractérisé en ce que

a) dans un composé de formule

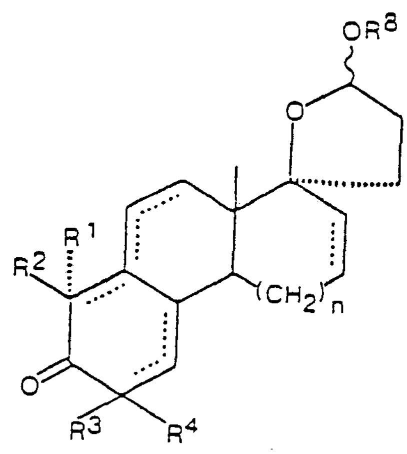

(II)

on oxyde le groupe $OR^8$ en groupe oxo ; ou bien

b) on soumet un composé de formule

(Voir figure, p. 32)

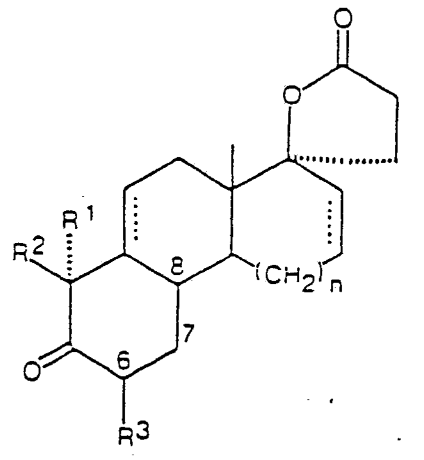

(Ia)

ou l'acide correspondant ou un sel de celui-ci à déshydrogénation en position 6 (7) ou 7 (8) ; ou bien

c) on halogène un composé de formule

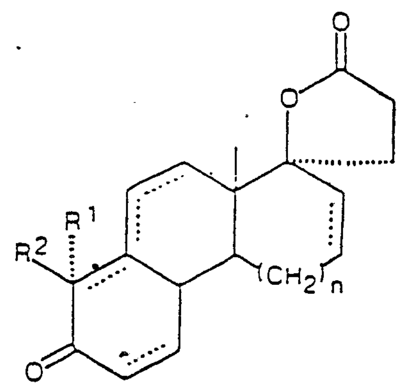

(Ib)

ou l'acide correspondant ou un sel de celui-ci ; ou bien

d) on fait réagir un composé de formule

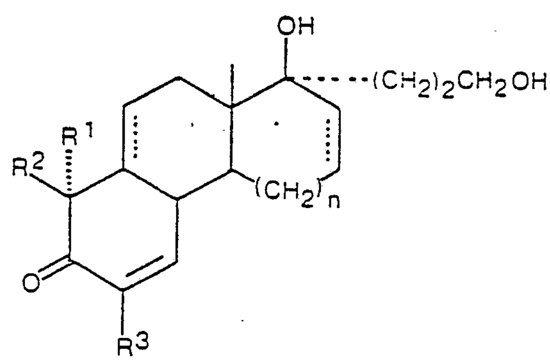

(Id)

avec un composé $R^7SH$, un alcanol ou C1-C5 ou le cyanure d'hydrogène ; ou bien

e) on soumet un composé de formule

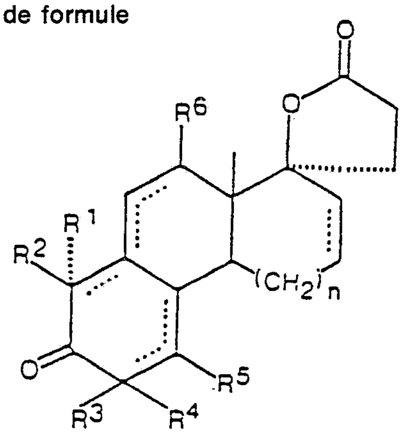

(Ih)

l'acide correspondant ou un sel de celui-ci à une réaction de Wittig avec un composé de méthylène-phosphonium ; ou bien

f) dans un composé de formule

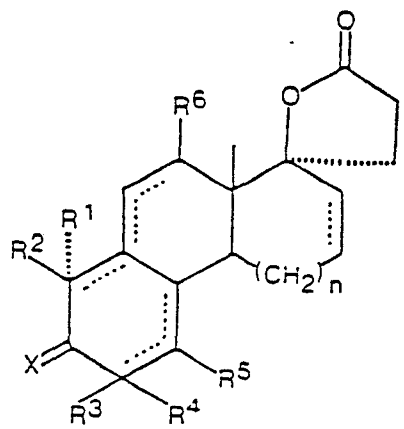

(Ii)

on ouvre le cycle lactone, ou bien

g) dans un composé de formule I dans laquelle Z représente le groupe COA, on réduit ce groupe en groupe hydroxyméthyle ; les symboles $R^1$, $R^2$, $R^3$, $R^4$, X, n et les liaisons cycliques en pointillé des formules ci-dessus ayant les significations déjà indiquées en $R^8$ représentant l'hydrogène ou un groupe alkyle en C1-C8.

**Revendications** (pour l'Etat contractant AT)

1. Procédé de préparation des dés-A-stéroïdes de formule

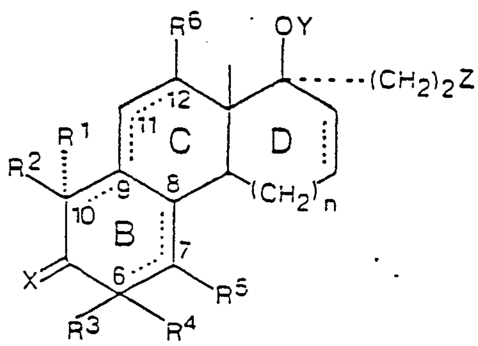

(IA)

dans laquelle $R^1$ et $R^2$ représentent l'hydrogène ou un groupe alkyle en $C_1$-$C_5$ ; $R^3$ représente l'hydrogène, un groupe méthyle ou un halogène, $R^4$ représente l'hydrogène ou bien, lorsque $R^3$ représente un groupe méthyle, l'hydrogène ou un groupe méthyle ; $R^5$ représente l'hydrogène, un groupe cyano, alcoxy en $C_1$-$C_5$ ou un reste $SR^7$ ; ou bien $R^3$ et $R^5$ forment ensemble un groupe méthylène ; $R^6$ représente l'hydrogène ou un groupe acylthio ; $R^7$ représente un groupe alcanoyle en $C_1$-$C_7$, alkyle en $C_1$-$C_8$, benzyle ou phénéthyle ; X représente un groupe oxo ou méthylène ; Y représente l'hydrogène ; Z représente un groupe hydroxyméthyle ou un groupe —COA ; A représente un groupe hydroxy ; ou bien Y et A forment ensemble une liaison O—C ; n est égal à 0 ou 1 et les liaisons cycliques figurant en pointillé sont facultatives ; chaque cycle contient au maximum une double liaison ; le cycle B est insaturé en 9 (10) lorsque le cycle C est insaturé en 11 (12) ; l'un au moins des symboles $R^5$ et $R^6$ représente l'hydrogène ; $R^5$ représente l'hydrogène lorsque $R^4$ représente l'hydrogène un groupe méthyle ; $R^5$ représente l'hydrogène ou bien $R^3$ et $R^5$ forment ensemble un groupe méthylène ; et $R^6$ représente l'hydrogène, lorsque Z représente un groupe carboxy ; $R^3$ représente l'hydrogène, un groupe méthyle ou un halogène et $R^4$ est absent lorsqu'il y a une double liaison 6 (7) ou 7 (8) ; la liaison 6 (7) et 11 (12) est saturée lorsque $R^6$ représente un groupe alcanoylthio en $C_1$-$C_7$ ; et les cycles B et C sont insaturés ; caractérisé en ce que

a) dans un composé de formule

(Voir figure, p. 34)

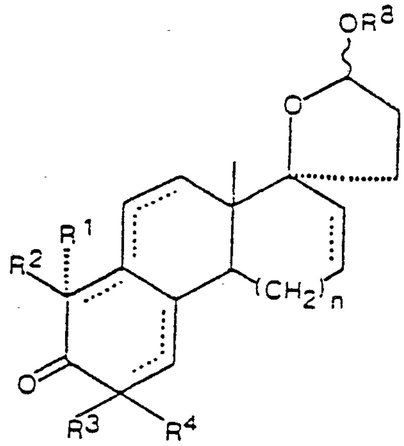

(II)

on oxyde le groupe $OR^8$ en groupe oxo ; ou bien

b) on soumet un composé de formule

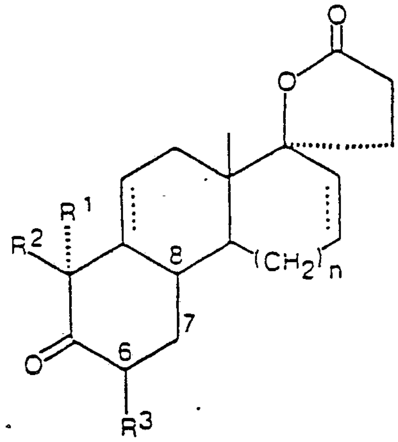

(Ia)

ou l'acide correspondant ou un sel de celui-ci à déshydrogénation en position 6 (7) ou 7 (8) ; ou bien

c) on soumet un composé de formule

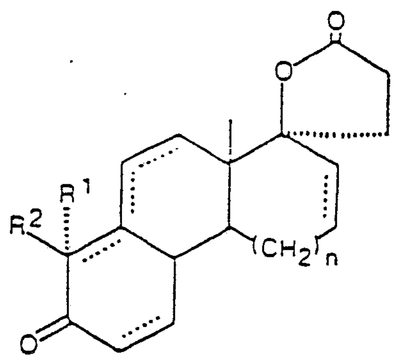

(Ib)

ou l'acide correspondant ou un sel de celui-ci, à halogénation ; ou bien

d) on fait réagir un composé de formule

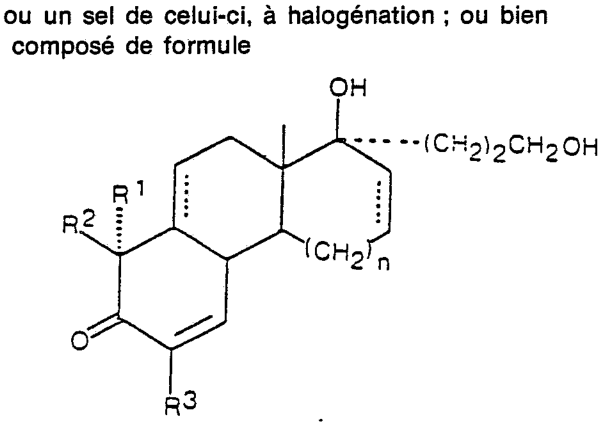

(Id)

avec un composé $R^7SH$, un alcanol en $C_1$-$C_5$ ou le cyanure d'hydrogène ; ou bien

e) on soumet un composé de formule

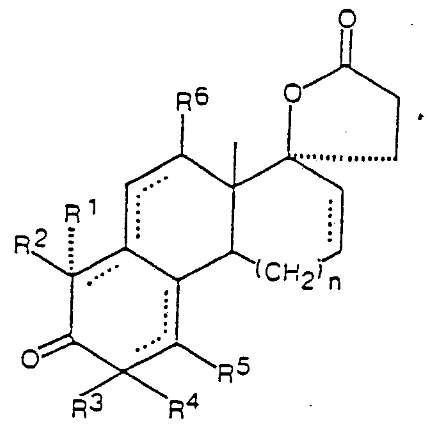

(Ih)

l'acide correspondant ou un sel de celui-ci à une réaction de Wittig avec un composé méthylène-phosphonium ; ou bien

f) dans un composé de formule

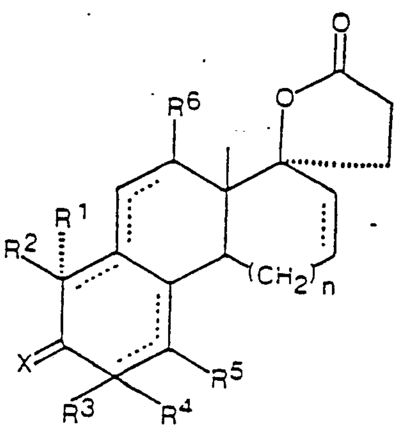

(Ii)

on ouvre le cycle lactone ; ou bien

g) dans un composé de formule I dans laquelle Z représente le groupe COA, on réduit ce groupe en groupe hydroxyméthyle ; les symboles $R^1$, $R^2$, $R^3$, $R^4$, X, n et les liaisons cycliques en pointillés des formules ci-dessus ayant les significations déjà indiquées et $R^8$ représentant l'hydrogène ou un groupe alkyle.

2. Procédé selon la revendication 1, caractérisé en ce que l'on prépare des dés-A-stéroïdes de formule

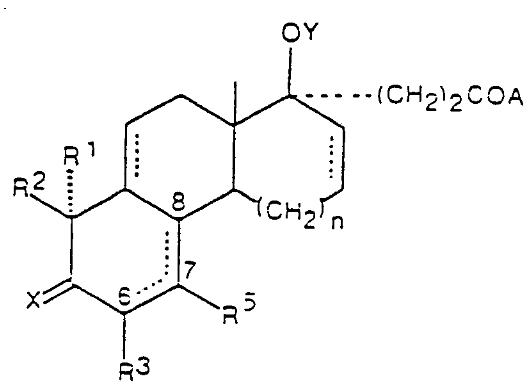

(IA-1)

dans laquelle $R^1$, $R^2$, $R^3$, $R^5$, A, Y, n et les liaisons en pointillés ont les significations indiquées dans la revendication 1.

3. Procédé selon les revendications 1 ou 2, caractérisé en ce que l'on prépare des dés-A-stéroïdes de formule IA ou I A-1 dans lesquelles au moins un des symboles $R^1$ et $R^2$ représentent un groupe alkyle en $C_1$-$C_5$.

4. Procédé selon les revendications 1, 2 ou 3, caractérisé en ce que l'on prépare des dés-A-stéroïdes de formule IA ou I A-1 dans lesquelles les cycles B et C sont insaturés.

5. Procédé selon l'une des revendications 1, 3 ou 4, caractérisé en ce que l'on prépare des dés-A-stéroïdes de formule Ia dans laquelle Z représente un groupe hydroxyméthyle.

6. Procédé selon la revendication 1, caractérisé en ce que l'on prépare la 5-oxo-A-tétranor-7α-pregna-9,11-diène-21,17-carbolactone.